# EUROPÄISCHE PATENTSCHRIFT

(11) **EP 1 551 810 B1**
(45) Veröffentlichungstag und Bekanntmachung des Hinweises auf die Patenterteilung: **30.11.2011**
(21) Anmeldenummer: 03771060.5
(22) Anmeldetag: 18.07.2003
(51) Int. Cl.: C07D 233/70, C07D 233/32, C07D 243/04, C07D 249/12, A61K 31/4166, A61P 3/04, A61P 3/10, C07D 401/12, C07D 209/02, C07D 491/10, C07D 403/12, C07D 405/12, C07D 409/12, C07D 417/12, C07D 487/08

(54) **DIARYLSUBSTITUIERTE CYCLISCHE HARNSTOFFDERIVATE MIT MCH-MODULATORISCHER WIRKUNG**
DIARYL-SUBSTITUTED CYCLIC UREA DERIVATIVES HAVING AN MCH-MODULATORY EFFECT
DERIVES D'UREE CYCLIQUES SUBSTITUES PAR DIARYLE, A EFFET MODULATEUR DE LA TCMH

(30) Priorität: 25.07.2002 DE 10233817
(43) Veröffentlichungstag der Anmeldung: 13.07.2005
(73) Patentinhaber: Sanofi-Aventis Deutschland GmbH, 65929 Frankfurt am Main (DE)
(72) Erfinder: SCHWINK, Lothar, 35260 Stadtallendorf (DE); STENGELIN, Siegfried, 65817 Eppstein (DE); GOSSEL, Matthias, 65719 Hofheim (DE); BOEHME, Thomas, 65428 Rüsselsheim (DE); HESSLER, Gerhard, 65719 Hofheim (DE); ROSSE, Gerard, Exton, PA 19341 (US); WALSER, Armin, Tuscon, AZ 85718 (US)
(86) Internationale Anmeldenummer: PCT/EP2003/007891
(87) Internationale Veröffentlichungsnummer: WO 2004/011438

(56) Entgegenhaltungen:
- EP-A- 0 503 548
- WO-A-02/10146
- WO-A-96/25410
- WO-A-03/057220
- WO-A1-98/58934
- NOLTE R J M ET AL.: "Host-guest complexation. 27. Hosts containing only cyclic urea binding sites " JOURNAL OF THE AMERICAN CHEMICAL SOCIETY., Bd. 106, Nr. 5, 1984, Seiten 1416-1420, XP002257972 AMERICAN CHEMICAL SOCIETY, WASHINGTON, DC., US ISSN: 0002-7863
- CRAM D J ET AL.: "Host-guest complexation. 31. A transacylase partial mimic" JOURNAL OF THE AMERICAN CHEMICAL SOCIETY., Bd. 106, Nr. 17, 1984, Seiten 4987-5000, XP002257973 AMERICAN CHEMICAL SOCIETY, WASHINGTON, DC., US ISSN: 0002-7863
- STEWART K D ET AL: "Host-guest complexation. 40. Synthesis and complexation of macrocyclic hosts containing cyclic ureas, anisyls, and steric barriers" JOURNAL OF ORGANIC CHEMISTRY, AMERICAN CHEMICAL SOCIETY. EASTON, US, Bd. 51, Nr. 23, 14. November 1986 (1986-11-14), Seiten 4327-4337, XP001120838 ISSN: 0022-3263
- SAITO Y ET AL: "Molecular characterization of the melanin-concentrating-hormone receptor", NATURE, NATURE PUBLISHING GROUP, LONDON, GB, vol. 400, no. 6741, 15 July 1999 (1999-07-15), pages 265-269, XP002152581, ISSN: 0028-0836, DOI: DOI:10.1038/22321
- CHAMBERS J ET AL: "MELANIN-CONCENTRATING HORMONE IS THE COGNATE LIGAND FOR THE ORPHAN G-PROTEIN-COUPLED RECEPTOR SLC-1", NATURE, NATURE PUBLISHING GROUP, LONDON, GB, vol. 400, 15 July 1999 (1999-07-15), pages 261-265, XP002947035, ISSN: 0028-0836, DOI: DOI:10.1038/22313

## Beschreibung

Die Erfindung betrifft substituierte Diarylheterocyclen sowie deren physiologisch verträgliche Salze und physiologisch funktionelle Derivate.

Es sind bereits den hier beschriebenen Diarylheterocyclen in ihrer Gesamtstruktur ähnliche Verbindungen mit pharmakologischer Wirkung im Stand der Technik beschrieben (wie zum Beispiel US006054590A).

Der Erfindung lag die Aufgabe zugrunde, Verbindungen zur Verfügung zu stellen, die eine Gewichtsreduktion bei Säugetieren bewirken und die zur Prävention und Behandlung von Obesitas geeignet sind.

Die Erfindung betrifft daher Verbindungen der Formel I, 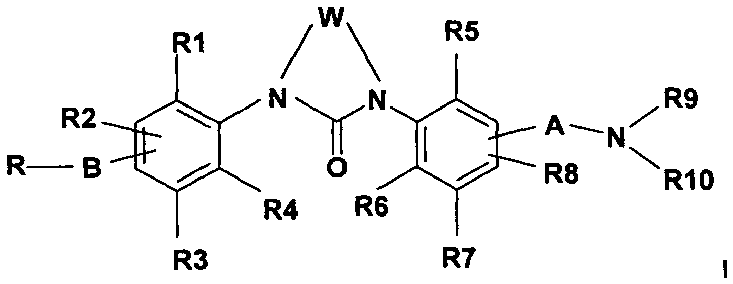 worin bedeuten
- R: (C₃-C₈)-Cycloalkyl; 5-6 gliedriger Ring, der ein oder zwei Heteroatome aus der Gruppe N, O und S enthalten kann und der 5-6 gliedrige Ring weitere Substituenten wie F, Cl, Br, NO₂, CF₃, OCF₃, CN, (C₁-C₆)-Alkyl, O-(C₁-C₆)-Alkyl tragen kann;
- B: O, S, CO, OCH₂, N(R23), CH₂;
- R23: H, (C₁-C₆)-Alkyl;
- R1, R2, R3 , R4: unabhängig voneinander H, F, Cl, Br, CF₃, O-(C₁-C₆)-Alkyl, (C₁-C₆)-Alkyl;
- W: -CH=CH-, -N=CH-;
- R5, R6, R7, R8: unabhängig voneinander H, F, Cl, Br, OH, CF₃, NO₂, CN, OCF₃, O-(C₁-C₆)-Alkyl
- A: eine Kette -(C(R42)(R43))ₘ -, in der ein Glied ersetzt sein kann durch ein Element aus der Gruppe O, N(R44);
- m: 3, 4;
- R42, R43, R44: unabhängig voneinander H, (C₁-C₆)-Alkyl, Aryl;
- R9, R10: unabhängig voneinander H, (C₁-C₈)-Alkyl, -(CH₂)ₒ -R45, CO-(C₁-C₈)-Alkyl; oder R9 und R10 bilden zusammen mit dem Stickstoffatom, an das sie gebunden sind, einen 4 bis 10-gliedrigen mono-, bi- oder spiro-cyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, (C₁-C₆)-Alkyl, O-(C₁-C₈)-Alkyl, oxo, CO(R46), CON(R47)(R48), Hydroxy, N(R50)CO(C₁-C₆)-Alkyl oder N(R51)(R52);
- R46, R47, R48, R50, R51, R52: unabhängig voneinander H, (C₁-C₆)-Alkyl;
- o: 0, 1, 2, 3, 4;
- R45: OH, 5-10 gliedriger mono- oder bicyclischer Ring, der ein oder zwei Heteroatome aus der Gruppe N, O und S enthalten kann und der 5-10 gliedrige Ring weitere Substituenten wie F, Cl, Br, OH, CF₃, oxo, O-(C₁-C₆)-Alkyl, (C₁-C₆)-Alky), (C₀-C₂)-Alkylen-Aryl, O-(C₀-C₂)-Alkylen-Aryl oder N(R51)(R52) enthalten kann;
sowie deren physiologisch verträglichen Salze.

Eine besondere Klasse innerhalb der besonders bevorzugten Verbindungen I bilden die Moleküle für die gilt
- W: -C=C-.

Eine weitere besondere Klasse innerhalb der besonders bevorzugten Verbindungen I bilden die Moleküle für die gilt
- m: 3;
- R42, R43, R44: H.

Eine weitere besondere Klasse innerhalb der besonders bevorzugten Verbindungen I bilden die Moleküle für die gilt
R5, R6, R7, R8 H.

Eine weitere besondere Klasse innerhalb der besonders bevorzugten Verbindungen I bilden die Moleküle in denen A und B jeweils in para-Position zum zentralen Heterozyklus angeordnet sind.

Können Reste oder Substituenten mehrfach in den Verbindungen der Formel I auftreten, wie zum Beispiel -(CH₂)ₒ -R45, so können sie alle unabhängig voneinander die angegebenen Bedeutungen haben und gleich oder verschieden sein.

Die Erfindung bezieht sich auf Verbindungen der Formel I, in Form ihrer Racemate, enantiomerenangereicherten Mischungen und reinen Enantiomere sowie auf ihre Diastereomere und Mischungen davon.

Die Alkyl-, Alkenyl- und Alkinylreste in den Substituenten R, R1, R2, R3, R4, R5, R6, R7, R8, R9, R10, R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44, R45, R46, R46, R47, R48, R49, R50, R51 und R52 können sowohl geradkettig, verzweigt oder optional halogeniert sein.

Unter dem Begriff "Aryl" wird eine Phenyl oder Naphthylgruppe verstanden.

Mono-, bi- oder spiro-cyclische Ringe können gesättigt, teilweise gesättigt oder ungesättigt und auch verbrückt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Benzolsulfon-, Benzoe-, Zitronen-, Methansulfon-, Fumar-, Glucon-, Glykol-, Isethion-, Milch-, Lactobion-, Malein-, Äpfel-, Methansulfon-, Bemstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, wie hierin beschrieben.

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wäßrigen oder.nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen. Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0;1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich sich durch ihre geringe Toxizität und ihre geringen Nebenwirkungen aus.

Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks. Die Verbindungen wirken als MCH Antagonisten und eignen sich auch zur Behandlung von Störungen des Empfindens und anderer psychiatrischen Indikationen, wie zum Beispiel Depressionen, Angstzuständen, Angstneurosen, Schizophrenie sowie zur Behandlung von Störungen assoziiert mit dem zirkadianen Rhythmus und zur Behandlung von Drogenmissbrauch.

Bei einem weiteren Aspekt der Erfindung können die Verbindungen der Formel I in Kombination mit einer oder mehreren weiteren pharmakologisch wirksamen Substanzen verabreicht werden, die beispielsweise ausgewählt sind aus Antidiabetika, Antiadiposita, blutdrucksenkenden Wirkstoffen, Lipidsenkern und Wirkstoffen zur Behandlung und/oder Prävention von Komplikationen, die von Diabetes verursacht werden oder mit Diabetes assoziiert sind.

Geeignete Antidiabetika umfassen Insuline, Amylin, GLP-1- und GLP-2-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanidine, Meglitinide, Oxadiazolidindione, Thiazolidindione, Glukosidase-Inhibitoren, Glukagon-Rezeptor-Antagonisten, GLP-1-Agonisten, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme verringern, PPAR- und RXR-Agonisten und Wirkstoffe, die auf den ATP-abhängigen Kaliumkanal der Betazellen wirken.

Bei einer Ausführungsform der Erfindung können die vorliegenden Verbindungen in Kombination mit Insulin verabreicht werden.

Bei einer weiteren Ausführungsform können die vorliegenden Verbindungen in Kombination mit einem Sulphonylharnstoff wie z.B. Tolbutamid, Glibenclamid, Glimepirid, Glipizid, Gliquidon, Glisoxepid, Glibornurid oder Gliclazid verabreicht werden.

Bei einer anderen Ausführungsform können die vorliegenden Verbindungen in Kombination mit einem Biguanid wie z.B. Metformin verabreicht werden.

Bei wieder einer anderen Ausführungsform können die vorliegenden Verbindungen in Kombination mit einem Meglitinid wie z.B. Repaglinid verabreicht werden.

Bei noch einer weiteren Ausführungsform können die vorliegenden Verbindungen in Kombination mit einem Thiazolidindion wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht werden.

Bei einer weiteren Ausführungsform können die vorliegenden Verbindungen in Kombination.mit einem α-Glukosidase-Inhibitor wie z.B. Miglitol oder Acarbose verabreicht werden.

Bei einer anderen Ausführungsform können die vorliegenden Verbindungen in Kombination mit einem Wirkstoff verabreicht werden, der auf den ATP-abhängigen Kaliumkanal der Betazellen wirkt, wie z.B. Tolbutamid, Glibenclamid, Glimepirid, Glipizid, Gliclazid oder Repaglinid.

Bei noch einer anderen Ausführungsform können die vorliegenden Verbindungen in Kombination mit einem antihyperlidämischen Wirkstoff oder einem antilipidämischen Wirkstoff wie z.B. Cholestyramin, Colestipol, Clofibrat, Fenofibrat, Gemfibrozil, Lovastatin, Pravastatin, Simvastatin, Atorvastatin, Cerivastatin, Fluvastafin, Probucol, Ezetimibe oder Dextrothyroxin verabreicht werden.

Bei einer weiteren Ausführungsform können die vorliegenden Verbindungen in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht werden. Weiterhin können die erfindungsgemäßen Verbindungen in Kombination mit einem oder mehreren Antiadiposita oder appetitregulierenden Wirkstoffen verabreicht werden.

Solche Wirkstoffe können ausgewählt werden aus der Gruppe bestehend aus CART-Agonisten, NPY-Antagonisten, MC4-Agonisten, Orexin-Antagonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Sertonin- und Noradrenalin-Wiederaufnahme-Inhibitoren, 5HT-Modulatoren, MAO-Hemmer, Bombesin-Agonisten, Galanin-Antagonisten, Wachstumshormon, Wachstumshormon freisetzende Verbindungen, TRH-Agonisten, Modulatoren der Entkopplungsproteine 2 oder 3, Leptin-Agonisten, Dopamin-Agonisten (Bromcriptin, Doprexin), Lipase/Amylase-Inhibitoren, Antagonisten des Cannabinoid Rezeptors 1, Modulatoren des die Acylierung stimulierende Protein (ASP), PPAR-Modulatoren, RXR-Modulatoren, hCNTF-Mimetika oder TR-β-Agonisten.

Bei einer Ausführungsform der Erfindung ist das Antiadipositum Leptin oder modifiziertes Leptin.

Bei einer anderen Ausführungsform ist das Antiadipositum Dexamphetamin oder Amphetamin.

Bei einer anderen Ausführungsform ist das Antiadipositum Fenfluramin oder Dexfenfluramin.

Bei noch einer anderen Ausführungsform ist das Antiadipositum Sibutramin oder die mono- und bisdemethylierten Wirkmetabolite von Sibutramin.

Bei einer weiteren Ausführungsform ist das Antiadipositum Orlistat.

Bei einer anderen Ausführungsform ist das Antiadipositum Mazindol, Diethylpropion oder Phentermin.

Weiterhin können die vorliegenden Verbindungen in Kombination mit einem oder mehreren antihypertensiven Wirkstoffen verabreicht werden. Beispiele für antihypertensive Wirkstoffe sind Betablocker wie Alprenolol, Atenol, Timolol, Pindolol, Propanolol und Metoprolol, ACE (Angiotensin Converting Enzym)-Hemmer wie z.B. Benazepril, Captopril, Enalapril, Fosinopril, Lisinopril, Quinapril und Rampril, Calciumkanal-Blocker wie Nifedipin, Felodipin, Nicardipin, Isradipin, Nimodipin, Diltiazem und Verapamil, sowie Alphablocker wie Doxazosin, Urapidil, Prazosin und Terazosin. Weiterhin kann verwiesen werden auf Remington: The Science and Practice of Pharmacy, 19. Auflage, Gennaro, Hrsg., Mack Publishing Co., Easton, PA, 1995.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

In zwei gleichzeitig erschienenen Artikeln in Nature (Nature 400, 261-264, 1999; Nature 400, 265-269, 1999, siehe Anlage) wurde separat von zwei Arbeitsgruppen ein hochspezifischer Rezeptor für das Melanin-Concentrating-Hormone (MCH) beschrieben. MCH übernimmt wichtige Funktionen bei der Steuerung der Nahrungsaufnahme. Verbindungen, die auf den MCH-Rezeptor wirken, besitzen daher eine anorektische Wirkung und sind zur Behandlung von Obesitas geeignet. Die Prüfung auf anorektische Wirkung der erfindungsgemäßen Verbindungen der Formel I wurde daher wie folgt durchgeführt.

### Funktionelle Messungen zur Ermittlung von IC50-Werten

Die Klonierung der cDNA für den humanen MCH-Rezeptor, Herstellung einer rekombinanten HEK293-Zellinie, welche den humanen MCH-Rezeptor exprimiert, sowie funktionelle Messungen mit der rekombinanten Zellinie erfolgten sinngemäß wie von Audinot et al. (J. Biol. Chem. 276,13554-13562, 2001) beschrieben. Im Unterschied zur Literaturstelle wurde jedoch für die Konstruktion des Expressionsvektors das Plasmid pEAK8 der Fa. EDGE Biosystems (USA) verwendet. Als Wirt für die Transfektion diente eine transformierte HEK-Zeilinie namens "PEAK Stable Cells" (ebenfalls von EDGE Biosystems). Die funktionellen Messungen des zellulären Calciumflusses nach Agonistenzugabe (MCH) in Gegenwart von erfindungsgemäßem Ligand erfolgten mit Hilfe des FLIPR-Gerätes der Fa. Molecular Devices (USA), unter Verwendung von Vorschriften des Geräteherstellers. Die Beispielverbindungen zeigten IC50-Werte in der Größenordnung von 0,01 bis >10 µM.

Die Wirksamkeit der Verbindungen wurde ferner wie folgt getestet:

### Biologisches Prüfmodell:

Die Prüfung der anorektischen Wirkung erfolgte an weiblichen NMRI Mäusen. Nach 17stündigem Futterentzug wurde über eine Schlundsonde das Testpräparat verabreicht. In Einzelhaltung und bei freiem Zugang zu Trinkwasser wurde den Tieren 30 Minuten nach Präparatgabe Kondensmilch angeboten. Der Kondensmilchverbrauch wurde halbstündlich 7 Stunden lang bestimmt und das Allgemeinbefinden der Tiere beobachtet. Der gemessene Milchverbrauch wurde mit den Vehikel-behandelten Kontrolltieren verglichen.

**Tabelle 1: Anorektische Wirkung, gemessen als Reduktion des kumulierten Milchkonsums behandelter im Vergleich zu Kontrolltieren.**

| Beispiel | Orale Dosis [mg/kg] | Anzahl der Tiere / Kumulierter Milchkonsum der behandelten Tiere N / [mL] | Anzahl der Tiere / Kumulierter Milchkonsum der Kontrolltiere N / [mL] | Reduktion des kumulierten Milchkonsum in % der Kontrolle |
|---|---|---|---|---|
| | | | | |
| Beispiel 1 | 30 | 5/1,7 | 5/4.1 | 58 |

Die nachfolgend aufgeführten Beispiele und Herstellungsmethoden dienen zur Erläuterung der Erfindung, ohne diese jedoch einzuschränken.

### Beispiel 1

### 1-[4-(2-Dimethylamino-ethoxy)-phenyl]-3-(4-phenoxy-phenyl)-1,3-dihydro-imidazol-2-on

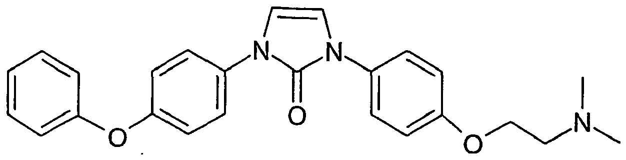

1-(2,2-Dimethoxy-ethyl)-3-[4-(2-dimethylamino-ethoxy)-phenyl]-1-(4-phenoxy-phenyl)-harnstoff (0,33 g) wurde mit Trifluoressigsäure (5 mL) für 16 Stunden geschüttelt. Flüchtige Anteile wurden entfernt und der Rückstand in Dichlormethan (10 mL) aufgenommen und mit Natronlauge (0,1 N; 1 mL) gewaschen. Die wässrige Phase wurde mit Dichlormethan (10 mL) extrahiert und die vereinigten organischen Phasen eingeengt. Der Rückstand wurde durch Chromatographie an Kieselgel gereinigt (Eluent: Dichlormethan/Methanol 98:2 mit 1 % v/v 7 N Ammoniaklösung in Methanol). Man erhielt so das Produkt mit dem Molekulargewicht 415,50 (C₂₅H₂₅N₃O₃); MS (ESI): 416 ([M+H]⁺).

### 1-(2,2-Dimethoxy-ethyl)-3-[4-(2-dimethylamino-ethoxy)-phenyl]-1-(4-phenoxy-phenyl)-harnstoff

Eine Lösung von Triphosgen (178 mg) in Chloroform (1 mL) wurde zu einer Lösung von 4-(2-Dimethylamino-ethoxy)-anilin (324 mg) in Chloroform (2 mL) und Pyridin (0,182 mL) bei 0 °C unter Stickstoff gegeben. Die Reaktion wurde dann bei Raumtemperatur für 30 Minuten geschüttelt. Die Lösung wurde dann zu einer Lösung von (2,2-Dimethoxy-ethyl)-(4-phenoxy-phenyl)-amin (410 mg) in Chloroform (2 mL) und Pyridin (0,182 mL) gegeben und für 3 Stunden bei 70 °C geschüttelt. Wasser (1 mL) wurde zugesetzt. Die Mischung wurde mit Dichlormethan (2 x 15 mL) extrahiert. Die vereinigten organischen Phasen wurden eingeengt und der Rückstand durch Chromatographie an Kieselgel gereinigt (Eluent: Dichlormethan/Methanol 98:2 mit 1 % v/v 7 N Ammoniaklösung in Methanol). Man erhielt so das Produkt mit dem Molekulargewicht 479,58 (C₂₇H₃₃N₃O₅); MS (ESI): 442 ([M-OMe]⁺).

### (2,2-Dimethoxy-ethyl)-(4-phenoxy-phenyl)-amin

Eine Lösung von 4-Phenoxyanilin (915 mg) in Dimethylformamid (5 mL) wurde mit Bromacetaldehyddimethylacetal (1,1 mL) und BEMP (1,9 mL) versetzt. Die Reaktionsmischung wurde für 16 Stunden bei 100 °C geschüttelt. Flüchtige Anteile wurden entfernt und der Rückstand durch Chromatographie an Kieselgel gereinigt (Eluent: Dichlormethan/Hexan 1:2, später 1:1 mit jeweils 1 % v/v 7 N Ammoniaklösung in Methanol). Man erhielt so das Produkt mit dem Molekulargewicht 273,33 (C₁₆H₁₉NO₃); MS (ESI): 274 ([M+H]⁺).

### Beispiel 2

### 1-[4-(2-Dimethylamino-ethylamino)-3-nitro-phenyl]-3-(4-phenoxy-phenyl)-1,3-dihydroimidazol-2-on

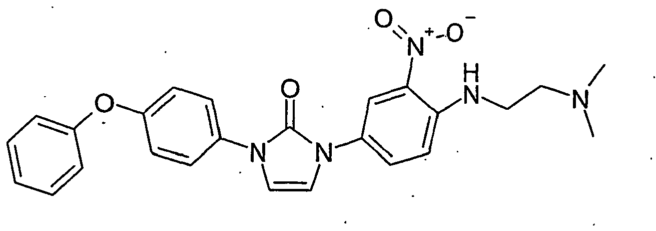

Eine Lösung von 1-(4-Fluor-3-nitro-phenyl)-3-(4-phenoxy-phenyl)-1,3-dihydro-imidazol-2-on (100 mg) in Dimethylformamid (2 mL) wurde mit 2-Dimethylamino-ethylamin (0,3 mL) und für 2 Stunden bei 70 °C geschüttelt. Flüchtige Anteile wurden entfernt und der Rückstand zwischen Dichlormethan und Wasser verteilt. Die organische Phase wurde getrocknet und eingeengt. Man erhielt so das Produkt mit dem Molekulargewicht 459,51 (C₂₅H₂₅N₅O₄); MS (ESI): 460 ([M+H]⁺).

1-(4-Fluor-3-nitro-phenyl}-3-(4-phenoxy-phenyl)-1,3-dihydro-imidazol-2-on Zu einer Lösung von (2,2-Dimethoxy-ethyl)-(4-phenoxy-phenyl)-amin (750 mg) in Chloroform (10 mL) wurde 4-Fluor-3-nitro-phenylisocyanat (0,45 mL) gegeben. Die Reaktion wurde für 2 Stunden bei 70 °C geschüttelt. Eine zweite Portion 4-Fluor-3-nitro-phenylisocyanat (0,45 mL) wurde zugesetzt. Nach 16 Stunden bei 70 °C wurden flüchtige Anteile entfernt und TFA (10 mL) zugesetzt. Nach 16 Stunden Schütteln wurden flüchtige Anteile entfernt und der Rückstand durch Chromatographie an Kieselgel (Eluent: Dichlormethan/Hexan 8:1) gereinigt. Man erhielt so das Produkt mit dem Molekulargewicht 391,36 (C₂₁H₁₄FN₃O₄); MS (ESI): 392 ([M+H]⁺).

### Beispiel 3

### 1-[3-Amino-4-(2-dimethylamino-ethylamino)-phenyl]-3-(4-phenoxy-phenyl)-1,3-dihydro-imidazol-2-on

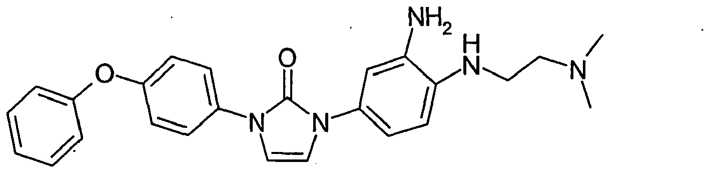

Eine Lösung von 1-[4-(2-Dimethylamino-ethylamino)-3-nitro-phenyl]-3-(4-phenoxyphenyl)-1,3-dihydro-imidazol-2-on (50 mg) in Dichlormethan (10 ml) und Eisessig (1 mL) wurde mit Zinkstaub (250 mg) versetzt. Nach 10 Minuten wurde von festen Anteilen abfiltriert und das Filtrat mit gesättigter Natriumcarbonatlösung gewaschen. Die organische Phase wurde über Magnesiumsulfat getrocknet und eingeengt. Man erhielt so das Produkt mit dem Molekulargewicht 429,53 (C₂₅H₂₇N₅O₂); MS (ESI): 430 ([M+H]⁺).

### Beispiel 5

### 1-[4-(2-Phenethylamino-ethoxy)-phenyl]-3-(4-phenoxy-phenyl)-1,3-dihydro-imidazol-2-on

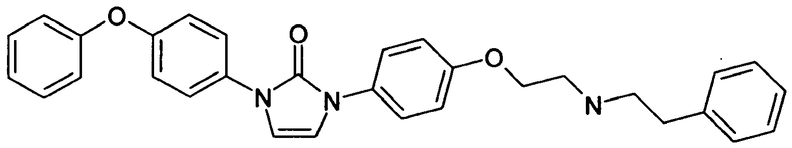

Eine Lösung von 1-[4-(2-Brom-ethoxy)-phenyl]-3-(4-phenoxy-phenyl)-1,3-dihydroimidazol-2-on (50 mg) in Acetonitril (1 mL) wurde mit Phenethylamin (47 mg), kaliumcarbonat (70 mg) und Natriumiodid (5 mg) versetzt und für 2 Stunden am Rückfluß gekocht. Die abgekühlte Lösung wurde filtriert und eingeengt. Der Rückstand wurde durch präparative HPLC gereinigt. Man erhielt so das Produkt mit dem Molekulargewicht 491,60 (C₃₁H₂₉N₃O₃); MS (ESI): 492 ([M+H]⁺) als Hydroformiat.

1-[4-(2-Brom-ethoxy)-phenyl]-3-(4-phenoxy-phenyl)-1,3-dihydro-imidazol-2-on Eine Lösung von 1-(4-Hydroxy-phenyl)-3-(4-phenoxy-phenyl)-1,3-dihydro-imidazol-2-on (4,7 g) in 1,2-Dibromethan (23,6 mL) wurde mit Natronlauge (3 N, 9,1 mL) und Tetrabutylammoniumhydrogensulfat (232 mg) versetzt. Die Mischung wurde für 2 Stunden auf 75 °C erwärmt. Nach dem Abkühlen wurde die Reaktionsmischung mit Dichlormethan verdünnt und mit Natronlauge (1 N), Salzsäure (1 N) und gesättigter Kochsalzlösung gewaschen. Die organische Phase wurde über Magnesiumsulfat getrocknet, eingeengt und der Rückstand durch Chromatographie an Kieselgel (Eluent: Dichlormethan/Methanol 9:1) gereinigt. Man erhielt so das Produkt mit dem Molekulargewicht 451,32 (C₂₃H₁₉N₂O₃); MS (ESI): 451 ([M+H]⁺).

### 1-(4-Hydroxy-phenyl)-3-(4-phenoxy-phenyl)-1,3-dihydro-imidazol-2-on

Eine Lösung von 1-(4-Methoxy-phenyl)-3-(4-phenoxy-phenyl)-1,3-dihydro-imidazol-2-on (6,0 g) in Dichlormethan (75 mL) wurde bei 0 °C mit Bortribromid (2,5 mL) versetzt. Nach 3 Stunden wurde gesättigte Natriumhydrogencarbonatlösung zugesetzt und die organische Phase mit gesättigter Kochsalzlösung gewaschen. Die organische Phase wurde über Magnesiumsulfat getrocknet, eingeengt und der Rückstand durch Chromatographie an Kieselgel (Eluent: Dichlormethan/Methanol 9:1) gereinigt. Man erhielt so das Produkt mit dem Molekulargewicht **344**,37 (C₂₁H₁₆N₂O₃); MS (ESI): 345 ([M+H]⁺).

1-(4-Methoxy-phenyl)-3-(4-phenoxy-phenyl)-1,3-dihydro-imidazol-2-on Eine Lösung von 1-(2,2-Dimethoxy-ethyl)-3-(4-methoxy-phenyl)-1-(4-phenoxy-phenyl)-harnstoff (6,8 g) in Trifluoressigsäure (20 mL) wurde 16 Stunden stehen gelassen und dann mit gesättigter Natriumcarbonatlösung neutralisiert. Die Mischung wurde mit Ethylacetat extrahiert. Die organische Phase wurde über Magnesiumsulfat getrocknet und eingeengt. Man erhielt so das Produkt mit dem Molekulargewicht 358,40 (C₂₂H₁₈N₂O₃); MS (ESI): 359 ([M+H]⁺).

### 1-(2,2-Dimethoxy-ethyl)-3-(4-methoxy-phenyl)-1-(4-phenoxy-phenyl)-harnstoff

Zu einer Lösung von Carbonyldiimidazol (2,51 g) in Dimethylformamid (15 mL) wurde bei 0 °C eine Lösung von 4-Methoxyanilin (1,9 g) in Dimethylformamid (3 mL) getropft. Nach 15 Minuten Reaktionszeit bei 0 °C wurde noch 45 Minuten bei Raumtemperatur reagieren gelassen. Dann wurde (2,2-Dimethoxy-ethyl)-(4-phenoxy-phenyl)-amin (4,1 g) in Dimethylformamid (2 mL) zugesetzt und für 2 Stunden auf 80 °C erwärmt. Nach dem Abkühlen wurden flüchtige Anteile entfernt und der Rückstand in Ethylacetat aufgenommen. Die organische Phase wurde mit Wasser und gesättigter Kochsalzlösung gewaschen. Die organische Phase wurde über Magnesiumsulfat getrocknet und eingeengt. Man erhielt so das Produkt mit dem Molekulargewicht 422,49 (C₂₄H₂₆N₂O₅); MS (ESI): 423 ([M+H]⁺).

### Beispiel 6

### 1-[4-(2-Methylamino-ethoxy)-phenyl]-3-(4-phenoxy-phenyl)-1,3-dihydro-imidazol-2-on

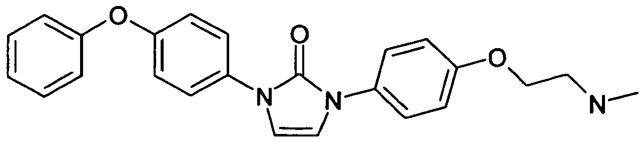

Eine Lösung von 1-[4-(2-Brom-ethoxy)-phenyl]-3-(4-phenoxy-phenyl)-1,3-dihydroimidazol-2-on (50 mg) in Acetonitril (1 mL) wurde mit Methylamin (1 M in THF, 1 mL) und Natriumiodid (5 mg) und 24 Stunden stehen gelassen. Die Reaktionslösung wurde filtriert und eingeengt. Der Rückstand wurde durch präparative HPLC gereinigt. Man erhielt so das Produkt mit dem Molekulargewicht 401,47 (C₂₄H₂₃N₃O₃); MS (ESI): 402 ([M+H]⁺) als Hydroformiat.

### Beispiel 7

### 1-[4-(2-Amino-ethoxy)-phenyl]-3-(4-phenoxy-phenyl)-1, 3-dihydro-imidazol-2-on

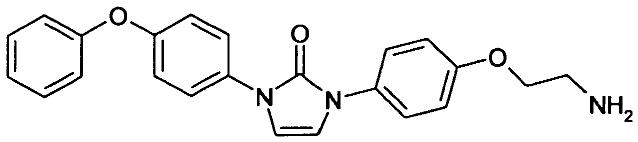

Eine Lösung von 1-[4-(2-Brom-ethoxy)-phenyl]-3-(4-phenoxy-phenyl)-1,3-dihydroimidazol-2-on (50 mg) in Acetonitril (1 mL) wurde mit Ammoniaklösung (1 mL) und Natriumiodid (5 mg) und 24 Stunden stehen gelassen. Nach wiederholter Zugabe der Ammoniaklösung wurde die Reaktion für 5 Stunden bei 40 °C gehalten. Die abgekühlte Reaktionslösung wurde filtriert und eingeengt. Der Rückstand wurde durch präparative HPLC gereinigt. Man erhielt so das Produkt mit dem Molekulargewicht 387,44 (C₂₄H₂₁N₃O₃); MS (ESI): 388 ([M+H]⁺) als Hydroformiat.

### Beispiel 8

### 1-(4-Cyclopentyloxy-phenyl)-3-[4-(3-dimethylamino-propoxy)-phenyl]-1,3-dihydroimidazol-2-on

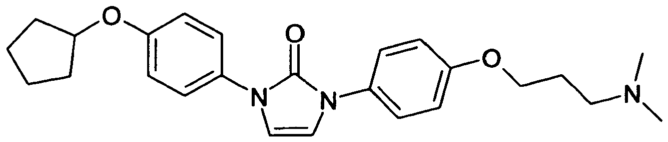

Ein Lösung von 4-(3-Dimethylamino-propoxy)-phenylamin (194 mg) in Dimethylformamid (5 mL) wurde bei 0 °C mit Carbonyldiimidazol (163 mg) versetzt. Nach 10 Minuten bei 0 °C und 30 Minuten bei Raumtemperatur wurde eine Lösung von (4-Cyclopentyloxy-phenyl)-(2,2-dimethoxy-ethyl)-amin (265 mg) in Dimethylformamid (1 mL) zugesetzt und die Mischung für 2 Stunden auf 80 °C erwärmt. Nach dem Abkühlen auf Raumtemperatur wurde Trifluoressigsäure (1 mL) zugesetzt. Nach 72 Stunden wurde mit Wasser verdünnt und mit Ethylacetat extrahiert. Die organische Phase wurde mit gesättigter Natriumhydrogencarbonatlösung gewaschen, getrocknet und eingeengt. Der Rückstand wurde durch präparative HPLC gereinigt. Man erhielt so das Produkt mit dem Molekulargewicht 421,54 (C₂₅H₃₁N₃O₃); MS (ESI): 422 ([M+H]⁺) als Hydrotrifluoracetat.

### 4-(3-Dimethylamino-propoxy)-phenylamin

Eine Lösung von Dimethyl-[3-(4-nitro-phenoxy)-propyl]-amin (3,75 g) in Ethanol (75 mL) wurde unter Argon mit Palladium(II)hydroxid (20% auf Kohle; 0,4 g) versetzt. Dann wurde Ameisensäure (4 mL) zugertopft wobei sich die Reaktionsmischung unter starker Gasentwicklung auf 60 °C erwärmte. Nach einer Reaktionszeit von 90 Minuten wurde der Katalysator abfiltriert und das Filtrat eingeengt. Der Rückstand wurde zwischen Methyl-tert.-butylether und Natronlauge (2 N) verteilt. Die organische Phase wurde getrocknet und eingeengt. Man erhielt so das Produkt mit dem Molekulargewicht 194,28 (C₁₁H₁₈N₂O); MS (ESI): 195 ([M+H]⁺).

### Dimethyl-[3-(4-nitro-phenoxy)-propyl]-amin

Eine Mischung aus 4-Fluornitrobenzol (2.82 g), 3-Dimethylaminopropanol (2,48 g), pulverisiertes Kaliumhydroxid (1,35 g) und Aliquat 336 wurde für eine Stunde auf 85 °C erwärmt. Das abgekühlte Rohgemisch wurde durch Chromatographie an Kieselgel (Eluent: Ethylaceat/Methanol 9:1 versetzt mit 1% v/v Triethylamin) gereinigt. Man erhielt so das Produkt mit dem Molekulargewicht 224,26 (C₁₁H₁₆N₂O₃); MS (ESI): 225 ([M+H]⁺).

### (4-Cyclopentyloxy-phenyl)-(2,2-dimethoxy-ethyl)-amin

Eine Suspension von 4-Cyclopentyloxy-phenylamin (8,86 g), Bromacetaldehyddimethylacetal (12,2 g), Kaliumcarbonat (13,8 g) und Dimethylformamid (100 mL) wurde für 5 Stunden auf 100 °C erhitzt. Nach dem Abkühlen wurde filtriert und das Filtrat eingeengt. Der Rückstand wurde durch Chromatographie an Kieselgel (Eluent: Heptan/Ethylacetat 4:1) gereinigt. Man erhielt so das Produkt mit dem Molekulargewicht 265,36 (C₁₅H₂₃NO₃); MS (ESI): 266 ([M+H]⁺).

### 4-Cyclopentyloxy-phenylamin

Eine Lösung von 4-Aminophenol (10,9 g) in Dimethylformamid (150 mL) wurde portionsweise mit Natriumhydrid (50%ig in Öl; 4,8 g) versetzt. Nach 20 Minuten wurde Cyclopentylbromid (14,9 g) zugetropft. Nach 2 Stunden bei Raumtemperatur wurde die Reaktionsmischung mit Wasser verdünnt und mit Ethylacetat extrahiert. Die organische Phase wurde mit Wasser und Kochsalzlösung gewaschen, getrocknet und eingeengt. Der Rückstand wurde durch Chromatographie an Kieselgel (Eluent: Heptan/Ethylacetat 3:1) gereinigt. Man erhielt so das Produkt mit dem Molekulargewicht 177,25 (C₁₁H₁₅NO); MS (ESI): 178 ([M+H]⁺).

### Beispiel 9

### 1-(4-Cyclopentyloxy-phenyl)-3-[4-((1R,2S)-2-dimethylamino-1-phenyl-propoxy)-phenyl]-1,3-dihydro-imidazol-2-on

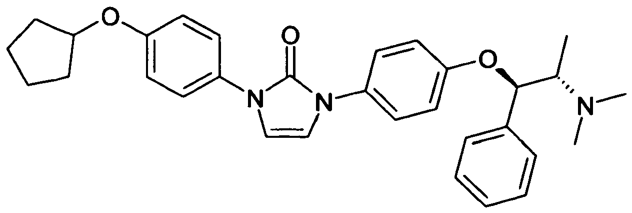

Die Verbindung wurde hergestellt wie im Beispiel 8 beschrieben nur unter Verwendung von 4-((1R,2S)-2-Dimethylamino-1-phenyl-propoxy)-phenylamin. Man erhielt so das Produkt mit dem Molekulargewicht 497,64 (C₃₁H₃₅N₃O₃); MS (ESI): 498 ([M+H]⁺) als Hydrotrifluoracetat.

### 4-((1R,2S)-2-Dimethylamino-1-phenyl-propoxy)-phenylamin

Die Verbindung wurde hergestellt wie im Beispiel 8 beschrieben nur unter Verwendung von (1 R,2S)-Dimethyl-[1-methyl-2-(4-nitro-phenoxy)-2-phenyl-ethyl]-amin. Man erhielt so das Produkt mit dem Molekulargewicht 270,38 (C₁₇H₂₂N₂O); MS (ESI): 271 ([M+H]⁺).

(1R,2S)-Dimethyl-[1-methyl-2-(4-nitro-phenoxy)-2-phenyl-ethyl]-amin Die Verbindung wurde hergestellt wie im Beispiel 8 beschrieben nur unter Verwendung von (1 R,2S)-Dimethyl-[1-methyl-2-hydroxy)-2-phenyl-ethyl]-amin. Man erhielt so das Produkt mit dem Molekulargewicht 300,36 (C₁₇H₂₀N₂O₃); MS (ESI): 301 ([M+H]⁺).

### Beispiel 10

### 1-(4-Cyclopentyloxy-phenyl)-3-{4-[(2-dimethylamino-ethyl)-methyl-amino]-phenyl}-1,3-dihydro-imidazol-2-on

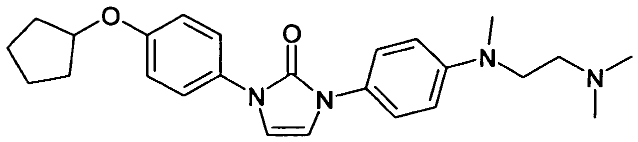

Die Verbindung wurde hergestellt wie im Beispiel 8 beschrieben nur unter Verwendung von N-(2-Dimethylamino-ethyl)-N-methyl-benzene-1,4-diamin. Man erhielt so das Produkt mit dem Molekulargewicht 420,56 (C₂₅H₃₂N₄O₂); MS (ESI): 421 ([M+H]⁺) als Hydrotrifluoracetat.

### N-(2-Dimethylamino-ethyl)-N-methyl-benzene-1,4-diamin

Die Verbindung wurde hergestellt durch Zinkstaubreduktion von N,N,N'-Trimethyl-N'-(4-nitro-phenyl)-ethan-1,2-diamin analog der Vorschrift im Beispiel 3. Man erhielt so das Produkt mit dem Molekulargewicht 193,29 (C₁₁H₁₉N₃); MS (ESI): 194 ([M+H]⁺).

### N,N,N'-Trimethyl-N'-(4-nitro-phenyl)-ethane-1,2-diamin

Eine Mischung aus N,N,N'-Trimethyl-ethan-1,2-diamin (2,17 g), Kaliumcarbonat (3,0 g), 4-Nitro-fluorbenzol und Dimethylformamid (30 mL) wurde für 72 Stunden gerührt. Die Reaktionsmischung wurde mit Wasser verdünnt und mit Ethylacetat extrahiert. Die organische Phase wurde mit Wasser und gesättigter Kochsalzlösung gewaschen, getrocknet und eingeengt. Man erhielt so das Produkt mit dem Molekulargewicht 223,28 (C₁₁H₁₇N₃O₂); MS (ESI): 224 ([M+H]⁺).

### Beispiel 11

### 1-(4-Cyclopentyloxy-phenyl)-3-[4-(3-dimethylamino-propyl)-phenyl]-1,3-dihydroimidazol-2-on

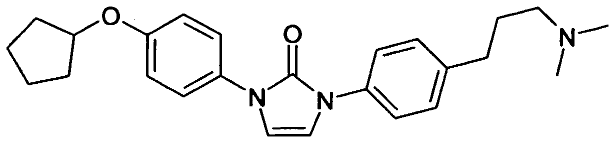

Die Verbindung wurde hergestellt wie im Beispiel 8 beschrieben nur unter Verwendung von 4-(3-Dimethylamino-propyl)-phenylamin. Man erhielt so das Produkt mit dem Molekulargewicht 405,54 (C₂₅H₃₁N₃O₂); MS (ESI): 406 ([M+H]⁺) als Hydrotrifluoracetat.

### 4-(3-Dimethylamino-propyl)-phenylamin

Eine Lösung von 3-Dimethylamino-1-(4-nitro-phenyl)-propan-1-on (Hydrochlorid, 0,25 g) in Eisessig (10 mL) wurde mit konzentrierter Salzsäure auf pH 1 gestellt und unter Stickstoff mit Palladium(II)hydroxid (20% auf Kohle, 0,1 g) versetzt. Die Stickstoffatomsphäre wurde durch Wasserstoff ersetzt und die Suspension für 6 Stunden geschüttelt. Es wurde vom Katalysator abfiltriert und das Filrat eingeengt. Der Rückstand wurde zwischen gesättigter Natriumhydrogencarbonatlösung und Ethylacetat verteilt. Die wässrige Phase wurde mit Natronlauge auf pH >12 gestellt und mit Ethylacetat extrahiert. Die vereinigten organischen Phasen wurden getrocknet und eingeengt. Man erhielt so das Produkt mit dem Molekulargewicht 178,28 (C₁₁H₁₈N₂); MS (ESI): 179 ([M+H]⁺).

### 3-Dimethylamino-1-(4-nitro-phenyl)-propan-1-on

Eine Mischung aus 4-Nitroacetophenon (1,0 g), N,N-Dimethylmethylenammoniumchlorid (0,56 g) und Acetonitril (10 mL) wurde für 4 Stunden auf 80 °C erhitzt und der nach dem Abkühlen ausgefallene Niederschlag abgesaugt. Man erhielt so das Produkt mit dem Molekulargewicht 222,25 (C₁₁H₁₄N₂O₃); MS (ESI): 223 ([M+H]⁺) als Hydrochlorid.

### Beispiele 12 - 91

Weitere Beispiele, die wie im Beispiel 5 beschrieben nur unter Verwendung des entsprechenden Amins hergestellt wurden, sind in der Tabelle 2 zusammengefasst

**Tabelle 2**

| Bsp. No. | Name | Struktur | Summen-formel | Molekular-gewicht | [M+H]⁺ ESI-MS |
|---|---|---|---|---|---|
| 12 | 1-{4-[2-(2-Methoxy-ethylamino)-ethoxy]-phenyl}-3-(4-phenoxy-phenyl)-1,3-dihydro-imidazol-2-on | 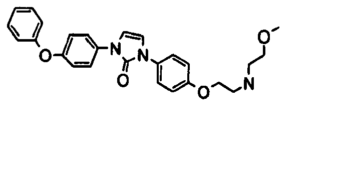 | C26H27N304 | 445,52 | 446 |
| 13 | 1-(4-Phenoxy-phenyl)-3-{4-[2-((1S,5R)-1,3,3-trimethyl-6-aza-bicyclo[3.2.1]oct-6-yl)-ethoxy]-phenyl}-1,3-dihydro-imidazol-2-on | 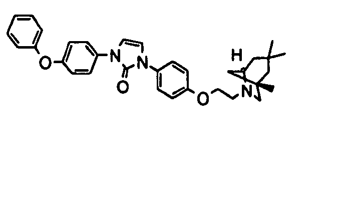 | C33H37N303 | 523,68 | 524 |
| 14 | 1-(4-Phenoxy-phenyl)-3-[4-(2-thiomorpholin-4-yl-ethoxy)-phenyl]-1,3-dihydro-imidazol-2-on | 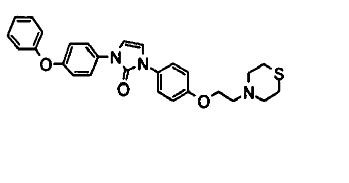 | C27H27N3O3S | 473,60 | 474 |
| 15 | 1-(2-{4-[2-Oxo-3-(4-phenoxy-phenyl)-2,3-dihydro-imidazol-1-yl]-phenoxy}-ethyl)-piperidine-3-carbonsäurediethylamid | 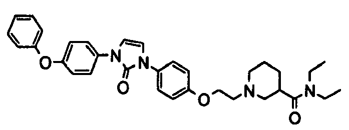 | C33H38N404 | 554,70 | 555 |
| 16 | 1-{4-[2-(1,4-Dioxa-8-aza-spiro[4.5]dec-8-yl)-ethoxy]-phenyl}-3-(4-phenoxy-phenyl)-1,3-dihydro-imidazol-2-on | 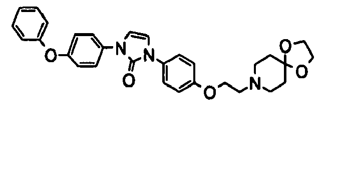 | C30H31N305 | 513,60 | 514 |
| 17 | 1-(4-{2-[Methyl-(2-pyridin-2-yl-ethyl)-amino]-ethoxy}-phenyl)-3-(4-phenoxy-phenyl)-1,3-dihydro-imidazol-2-on | 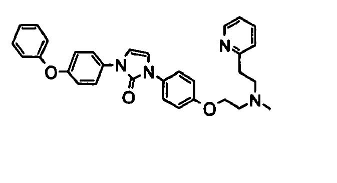 | C31H30N403 | 506,61 | 507 |
| 18 | 1-(4-Phenoxy-phenyl)-3-{4-[2-(4-phenyl-butylamino)-ethoxy]-phenyl}-1,3-dihydro-imidazol-2-on | 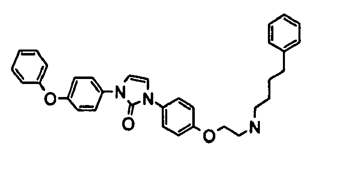 | C33H33N303 | 519,65 | 520 |
| 21 | 1-{4-[2-(2-Cyclohex-1-enyl-ethylamino)-ethoxy]-phenyl}-3-(4-phenoxy-phenyl)-1,3-dihydro-imidazol-2-on | 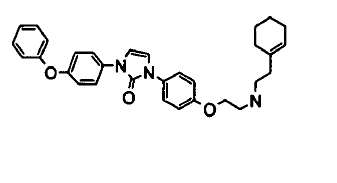 | C31H33N303 | 495,63 | 496 |
| 23 | (S)-3-(2-{4-[2-Oxo-3-(4-phenoxy-phenyl)-2,3-dihydro-imidazol-1-yl]-phenoxy}-ethylamino)-azepan-2-on | 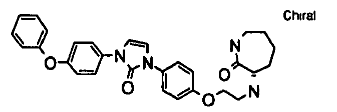 | C29H30N404 | 498,59 | 499 |
| 24 | 1-{4-[2-(4-Acetyl-piperazin-1-yl)-ethoxy]-phenyl}-3-(4-phenoxy-phenyl)-1,3-dihydro-imidazol-2-on | 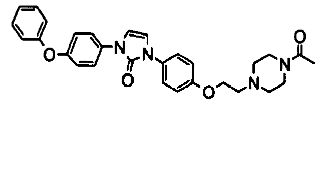 | C29H30N404 | 498,59 | 499 |
| 25 | 1-(4-{2-[3-(2-Oxo-pyrrolidin-1-yl)-propylamino]-ethoxy}-phenyl)-3-(4-phenoxy-phenyl)-1,3-dihydro-imidazol-2-on | 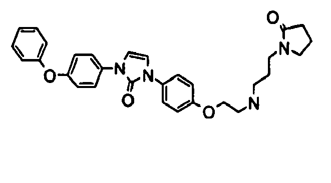 | C30H32N404 | 512,61 | 513 |
| 27 | 1-(4-Phenoxy-phenyl)-3-(4-{2-[(tetrahydro-furan-2-ylmethyl)-amino]-ethoxy}-phenyl)-1,3-dihydro-imidazol-2-on | 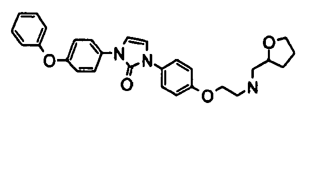 | C28H29N304 | 471,56 | 472 |
| 28 | 1-{4-[2-(4-Hydroxy-piperidin-1-yl)-ethoxy]-phenyl}-3-(4-phenoxy-phenyl)-1,3-dihydro-imidazol-2-on | 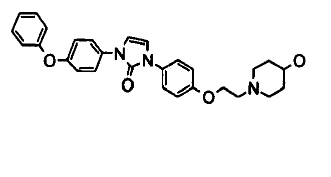 | C28H29N304 | 471,56 | 472 |
| 29 | 1-{4-[2-(3,4-Dihydro-1H-isochinolin-2-yl)-ethoxy]-phenyl}-3-(4-phenoxy-phenyl)-1,3-dihydro-imidazol-2-on | 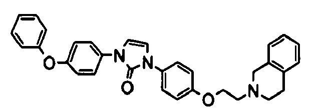 | C32H29N303 | 503,61 | 504 |
| 30 | 1-{4-[2-(4-Methyl-piperidin-1-yl)-ethoxy]-phenyl}-3-(4-phenoxy-phenyl)-1,3-dihydro-imidazol-2-on | 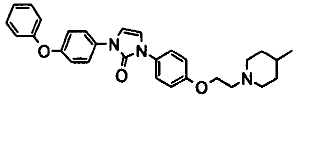 | C29H31N3O3 | 469,59 | 470 |
| 32 | 1-[4-(2-Phenethylamino-ethoxy)-phenyl]-3-(4-phenoxy-phenyl)-1,3-dihydro-imidazol-2-on | 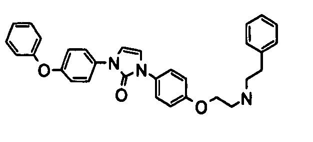 | C31H29N303 | 491,60 | 492 |
| 33 | 1-{4-[2-(Benzyl-methyl-amino)-ethoxy]-phenyl}-3-(4-phenoxy-phenyl)-1,3-dihydro-imidazol-2-on | 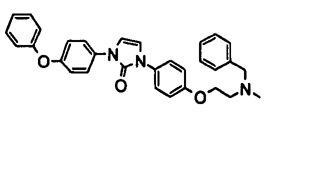 | C31H29N303 | 491,60 | 492 |
| 34 | 1-(4-Phenoxy-phenyl)-3-(4-{2-[(thiophen-2-ylmethyl)-amino]-ethoxy}-phenyl)-1,3-dihydro-imidazol-2-on | 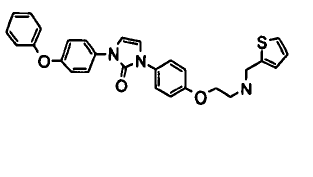 | C28H25N3O3S | 483,59 | 484 |
| 36 | 1-(4-Phenoxy-phenyl)-3-[4-(2-thiazolidin-3-yl-ethoxy)-phenyl]-1,3-dihydro-imidazol-2-on | 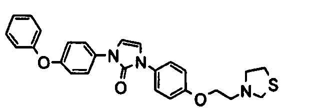 | C26H25N3O3S | 459,57 | 460 |
| 37 | 1-{4-[2-(Methyl-phenethyl-amino)-ethoxy]-phenyl}-3-(4-phenoxy-phenyl)-1,3-dihydro-imidazol-2-on | 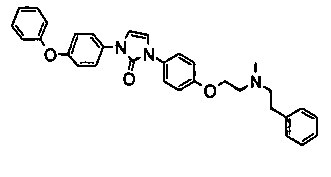 | C32H31N303 | 505,62 | 506 |
| 39 | 1-[4-(2-Cyclohexylamino-ethoxy)-phenyl]-3-(4-phenoxy-phenyl)-1,3-dihydro-imidazol-2-on | 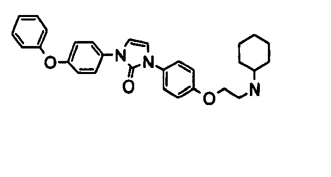 | C29H31N303 | 469, 59 | 470 |
| 40 | 1-[4-(2-tert-Butylamino-ethoxy)-phenyl]-3-(4-phenoxy-phenyl)-1,3-dihydro-imidazol-2-on | 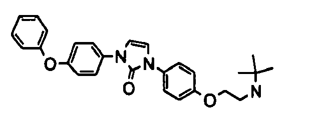 | C27H29N303 | 443,55 | 444 |
| 41 | 1-(4-Phenoxy-phenyl)-3-{4-[2-(2-pyridin-4-yl-ethylamino)-ethoxy]-phenyl}-1,3-dihydro-imidazol-2-on | 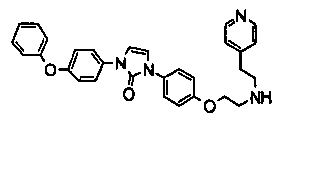 | C30H28N403 | 492,58 | 493 |
| 42 | 1-[4-(2-Isopropylamino-ethoxy)-phenyl]-3-(4-phenoxy-phenyl)-1,3-dihydro-imidazol-2-on | 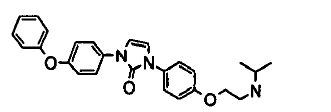 | C26H27N303 | 429,52 | 430 |
| 43 | N-[1-(2-{4-[2-Oxo-3-(4-phenoxy-phenyl)-2,3-dihydro-imidazol-1-yl]-phenoxy}-ethyl)-pyrrolidin-3-yl]-acetamid | 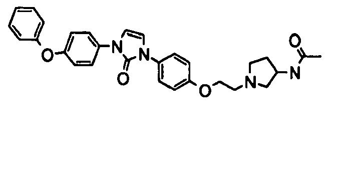 | C29H30N404 | 498,59 | 499 |
| 45 | 1-[4-(2-Imidazol-1-yl-ethoxy)-phenyl]-3-(4-phenoxy-phenyl)-1,3-dihydro-imidazol-2-on | 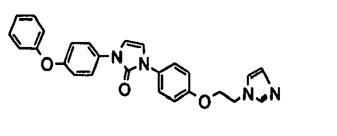 | C26H22N403 | 438,49 | 439 |
| 46 | 1-(4-Phenoxy-phenyl)-3-[4-(2-pyrazol-1-yl-ethoxy)-phenyl]-1,3-dihydro-imidazol-2-on | 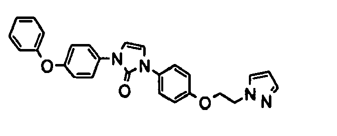 | C26H22N403 | 438,49 | 439 |
| 48 | 1-{4-[2-(Indan-2-ylamino)-ethoxy]-phenyl}-3-(4-phenoxy-phenyl)-1,3-dihydro-imidazol-2-on | 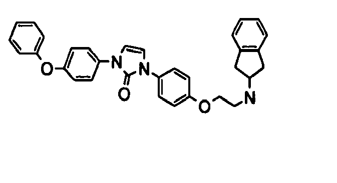 | C32H29N303 | 503,61 | 504 |
| 49 | 1-{4-[2-(2-Ethyl-piperidin-1-yl)-ethoxy]-phenyl}-3-(4-phenoxy-phenyl)-1,3-dihydro-imidazol-2-on | 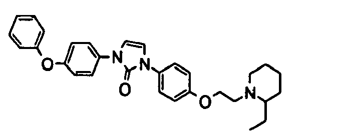 | C30H33N303 | 483,62 | 484 |
| 51 | 1-{4-[2-(Indan-1-ylamino)-ethoxy]-phenyl}-3-(4-phenoxy-phenyl)-1,3-dihydro-imidazol-2-on | 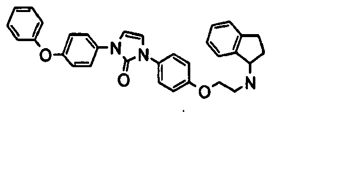 | C32H29N303 | 503,61 | 504 |
| 54 | 1-[4-(2-Benzoimidazol-1-yl-ethoxy)-phenyl]-3-(4-phenoxy-phenyl)-1,3-dihydro-imidazol-2-on | 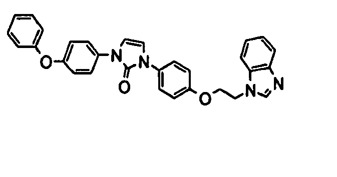 | C30H24N403 | 488,55 | 489 |
| 55 | 1-(4-{2-[2-(3-Methoxy-phenyl)-ethylamino]-ethoxy}-phenyl)-3-(4-phenoxy-phenyl)-1,3-dihydro-imidazol-2-on | 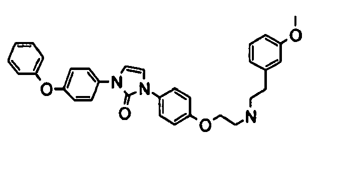 | C32H31N304 | 521,62 | 522 |
| 56 | 1-{4-[2-(2-Methyl-4,5-dihydro-imidazol-1-yl)-ethoxy]-phenyl}-3-(4-phenoxy-phenyl)-1,3-dihydro-imidazol-2-on | 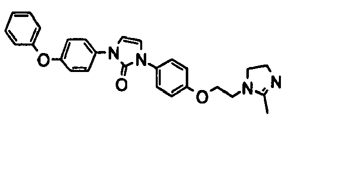 | C27H26N403 | 454,53 | 455 |
| 57 | 1-{4-[2-(4-Ethyl-piperazin-1-yl)-ethoxy]-phenyl}-3-(4-phenoxy-phenyl)-1,3-dihydro-imidazol-2-on | 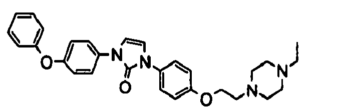 | C29H32N403 | 484,60 | 485 |
| 58 | 1-{4-[2-(3,6-Dihydro-2H-pyridin-1-yl)-ethoxy]-phenyl}3-(4-phenoxy-phenyl)-1,3-dihydro-imidazol-2-on | 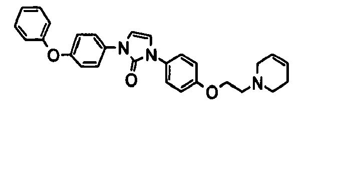 | C28H27N303 | 453,55 | 454 |
| 59 | 1-{4-[2-(3-Hydroxy-pyrrolidin-1-yl)-ethoxy]-phenyl}-3-(4-phenoxy-phenyl)-1,3-dihydro-imidazol-2-on | 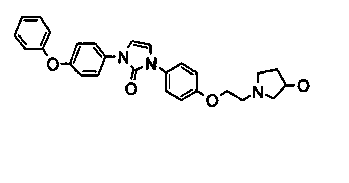 | C27H27N304 | 457,53 | 458 |
| 61 | 1-{4-[(4aR,8aS)-2-(Octahydro-isochinolin-2-yl)-ethoxy]-phenyl}-3-(4-phenoxy-phenyl)-1,3-dihydro-imidazol-2-on | 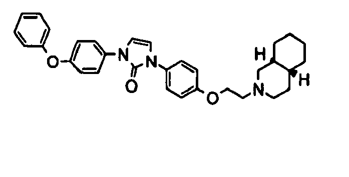 | C32H35N303 | 509,65 | 510 |
| 62 | 1-{4-[(4aS,8aS)-2-(Octahydro-isochinolin-2-yl)-ethoxy]-phenyl}-3-(4-phenoxy-phenyl)-1,3-dihydro-imidazol-2-on | 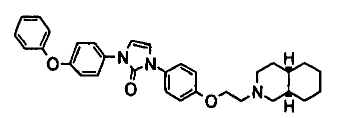 | C32H35N303 | 509,65 | 510 |
| 63 | 1-[4-(2-{[2-(1H-Indol-3-yl)-ethyl]-methyl-amino}-ethoxy)-phenyl]-3-(4-phenoxy-phenyl)-1,3-dihydro-imidazol-2-on | 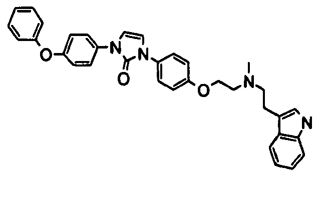 | C34H32N403 | 544,66 | 545 |
| 64 | 1-(4-{2-[2-(2-Chlor-phenyl)-ethylamino]-ethoxy}-phenyl)-3-(4-phenoxy-phenyl)-1,3-dihydro-imidazol-2-on | 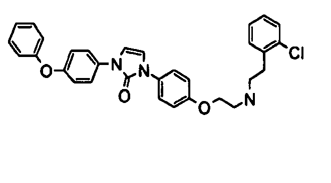 | C31 H28ClN3O3 | 526,04 | 526 |
| 65 | 1-(4-{2-[(5-Chlor-thiophen-2-ylmethyl)-amino]-ethoxy}-phenyl)-3-(4-phenoxy-phenyl)-1,3-dihydro-imidazol-2-on | 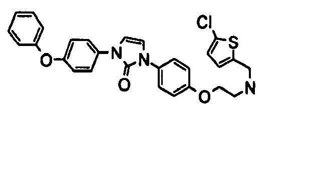 | C28H24ClN3O3 S | 518,04 | 518 |
| 66 | 1-{4-[2-((1R,2R)-2-Benzyloxy-cyclopentylamino)-ethoxy]-phenyl}-3-(4-phenoxy-phenyl)-1,3-dihydro-imidazol-2-on | 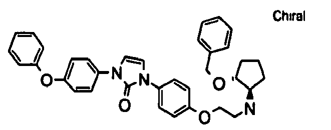 | C35H35N304 | 561,69 | 562 |
| 67 | 1-{4-[2-((S)-8-Methoxy-1,2,3,4-tetrahydro-naphthalen-2-ylamino)-ethoxy]-phenyl}-3-(4-phenoxy-phenyl)-1,3-dihydro-imidazol-2-on | 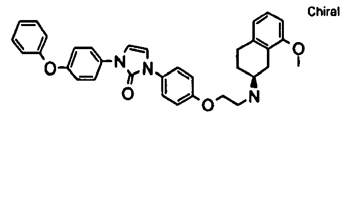 | C34H33N304 | 547,66 | 548 |
| 69 | 1-{4-[2-(5-Brom-2,3-dihydro-indol-1-yl)-ethoxy]-phenyl}-3-(4-phenoxy-phenyl)-1,3-dihydro-imidazol-2-on | 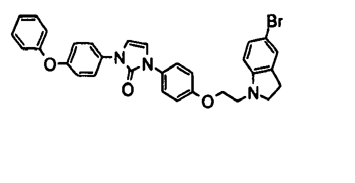 | C31 H26BrN3O3 | 568,48 | 568 |
| 70 | 1-{4-[2-(5-Ethyl-2-methyl-piperidin-1-yl)-ethoxy]-phenyl}-3-(4-phenoxy-phenyl)-1,3-dihydro-imidazol-2-on | 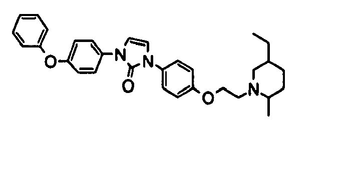 | C31H35N303 | 497,64 | 498 |
| 71 | 1-{4-[2-(2,5-Dimethyl-pyrrolidin-1-yl)-ethoxy]-phenyl}-3-(4-phenoxy-phenyl)-1,3-dihydro-imidazol-2-on | 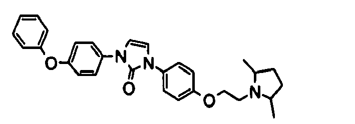 | C29H31N303 | 469,59 | 470 |
| 72 | 1-{4-[2-(Methyl-propyl-amino)-ethoxy]-phenyl}-3-(4-phenoxy-phenyl)-1,3-dihydro-imidazol-2-on | 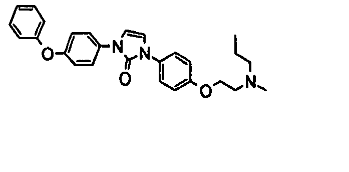 | C27H29N303 | 443,55 | 444 |
| 73 | 1-{4-[2-([1,3]Dioxolan-2-ylmethyl-methyl-amino)-ethoxy]-phenyl}-3-(4-phenoxy-phenyl)-1,3-dihydro-imidazol-2-on | 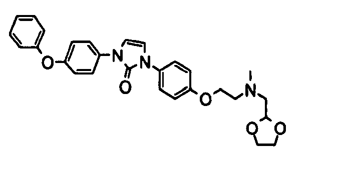 | C28H29N305 | 487,56 | 488 |
| 74 | 1-{4-[2-(2-Methyl-pyrrolidin-1-yl)-ethoxy]-phenyl}-3-(4-phenoxy-phenyl)-1,3-dihydro-imidazol-2-on | 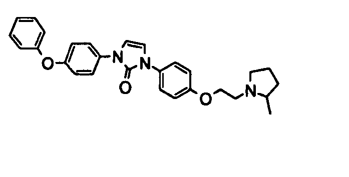 | C28H29N303 | 455,56 | 456 |
| 75 | 1-{4-[2-(Isopropyl-methyl-amino)-ethoxy]-phenyl}-3-(4-phenoxy-phenyl)-1,3-dihydro-imidazol-2-on | 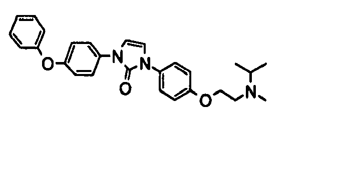 | C27H29N303 | 443,55 | 444 |
| 77 | (S)-1-(2-{4-[2-Oxo-3-(4-phenoxy-phenyl)-2,3-dihydro-imidazol-1-yl]-phenoxy}-ethyl)-pyrrolidine-2-carbonsäuredimethylamid | 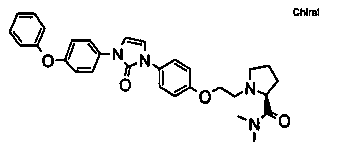 | C30H32N404 | 512,61 | 513 |
| 79 | 1-{4-[2-(7-Aza-bicyclo[2.2.1]hept-7-yl)-ethoxy]-phenyl}-3-(4-phenoxy-phenyl)-1,3-dihydro-imidazol-2-on | 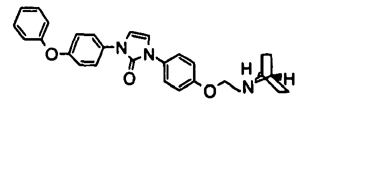 | C29H29N303 | 467,57 | 468 |
| 80 | 1-(4-{2-[(1-Benzyl-pyrrolidin-3-yl)-methyl-amino]-ethoxy}-phenyl)-3-(4-phenoxy-phenyl)-1,3-dihydro-imidazol-2-on | 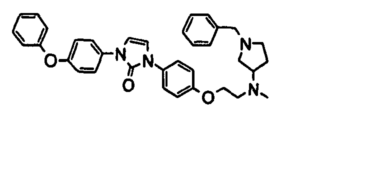 | C35H36N403 | 560,70 | 561 |
| 81 | 1-(4-{2-[(1,1-Dioxo-tetrahydro-1lambda6-thiophen-3-yl)-methyl-amino]-ethoxy}-phenyl)-3-(4-phenoxy-phenyl)-1,3-dihydro-imidazol-2-on | 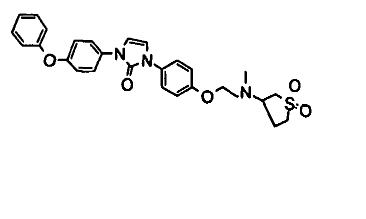 | C28H29N3O5S | 519,62 | 520 |
| 83 | 1-(4-{2-[((4S,5S)-2,2-Dimethyl-4-phenyl-[1,3]dioxan-5-yl)-methyl-amino]-ethoxy}-phenyl)-3-(4-phenoxy-phenyl)-1,3-dihydro-imidazol-2-on | 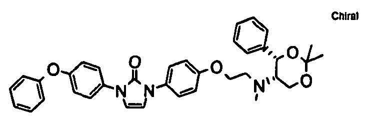 | C36H37N305 | 591,71 | 592 |
| 84 | 1-{4-[2-(3-Dimethylamino-pyrrolidin-1-yl)-ethoxy]-phenyl}-3-(4-phenoxy-phenyl)-1,3-dihydro-imidazol-2-on | 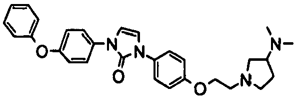 | C29H32N403 | 484,60 | 485 |

### Beispiel 92

### 1-(4-sec-Butoxy-phenyl)-3-[4-(2-dimethylamino-ethoxy)-phenyl]-1,3-dihydro-imidazol-2-on

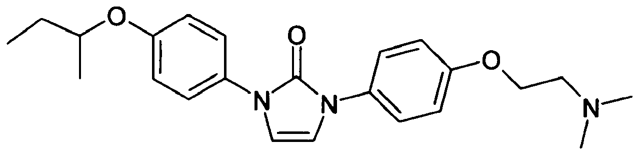

Eine Lösung von 1-[4-(2-Dimethylamino-ethoxy)-phenyl]-3-(4-hydroxy-phenyl)-1,3-dihydro-imidazol-2-on (50 mg) in Propionitril (1 mL) wurde mit Cäsiumcarbonat (100 mg) und 2-Butylbromid (25 mg) versetzt und für 2 Stunden auf 80 °C erwärmt. Die Reaktionslösung wurde filtriert und der eingeengt. Der Rückstand wurde durch präparative HPLC gereinigt. Man erhielt so das Produkt mit dem Molekulargewicht 395,51 (C₂₃H₂₉N₃O₃); MS (ESI): 396 ([M+H]⁺) als Hydrotrifluoracetat.

1-[4-(2-Dimethylamino-ethoxy)-phenyl]-3-(4-hydroxy-phenyl)-1,3-dihydro-imidazol-2-on Eine Suspension aus 1-(4-Benzyloxy-phenyl)-1-(2,2-dimethoxy-ethyl)-3-[4-(2-dimethylamino-ethoxy)-phenyl]-harnstoff (4,9 g), Palladium (10% auf Kohle, 1,0 g) und Ethanol (40 mL) wurde für 5 Stunden unter Wasserstoff gerührt. Es wurde vom Katalysator abfiltriert und das Filtrat eineengt. Der Rückstand wurde in Trifluoressigsäure (20 mL) aufgenommen und die Lösung für 48 Stunden gerührt. Die Reaktionsmischung wurde mit Dichlormethan verdünnt und mit gesättigter Natriumhydrogencarbonatlösung gewaschen. Die organische Phase wurde getrocknet und eingeengt. Der feste Rückstand wurde mit Acetonitril verrührt und das Produkt abfiltriert. Man erhielt so das Produkt mit dem Molekulargewicht 339,40 (C₁₉H₂₁N₃O₃); MS (ESI): 340 ([M+H]⁺).

### 1-(4-Benzyloxy-phenyl)-1-(2,2-dimethoxy-ethyl)-3-[4-(2-dimethylamino-ethoxy)-phenyl]-harnstoff

Zu einer eisgekühlten Lösung von 4-(2-Dimethylamino-ethoxy)-phenylamin (3,01 g) in Dimethylformamid (40 mL) wurde Carbonyldiimidazol (2,7 g) gegeben. Nach 30 Minuten wurde (4-Benzyloxy-phenyl)-(2,2-dimethoxy-ethyl)-amin (4,8 g) zugesetzt und die Mischung für 30 Minuten auf 80 °C erhitzt. Nach dem Abkühlen wurden flüchtige Anteile entfernt und der Rückstand durch MPLC (Eluent: Heptan/Ethylacetat 9:1) gereingt. Man erhielt so das Produkt mit dem Molekulargewicht 493,61 (C₂₈H₃₅N₃O₅), MS (ESI): 494 ([M+H]⁺).

### (4-Benzyloxy-phenyl)-(2,2-dimethoxy-ethyl)-amin

Eine Mischung aus 4-Benzyloxyanilin (10 g), Bromaceataldehyddimethylacetal (12,2 g), Kaliumcarbonat (13,8 g) und Dimethylformamid (150 mL) wurde für 5 Stunden auf 100 °C erwärmt. Nach dem Abkühlen wurde die Reaktionslösung filtriert und eingeengt. Der Rückstand wurde durch MPLC (Eluent: Heptan/Ethylacetat 4:1) gereinigt. Man erhielt so das Produkt mit dem Molekulargewicht 287,36 (C₁₇H₂₁NO₃); MS (ESI): 288 ([M+H]⁺).

### Beispiele 93 - 130

Weitere Beispiele, die wie im Beispiel 92 beschrieben unter Verwendung des entsprechenden Alkylbromids hergestellt wurden, sind in der Tabelle 3 zusammengefasst.

**Tabelle 3**

| Bsp. No. | Struktur | Name | Summen-formel | Molekular-gewicht | [M+H]⁺ ESI-MS |
|---|---|---|---|---|---|
| 97 | 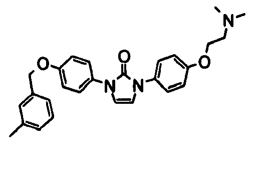 | 1-[4-(2-Dimethylamino-ethoxy)-phenyl]-3-[4-(3-methyl-benzyloxy)-phenyl]-1,3-dihydro-imidazol-2-on | C27H29N303 | 443,55 | 444 |
| 98 | 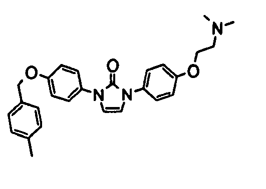 | 1-[4-(2-Dimethylamino-ethoxy)-phenyl]-3-[4-(4-methyl-benzyloxy)-phenyl]-1,3-dihydro-imidazol-2-on | C27H29N303 | 443,55 | 444 |
| 111 | 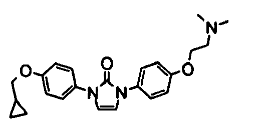 | 1-(4-Cyclopropylmethoxy-phenyl)-3-[4-(2-dimethylamino-ethoxy)-phenyl]-1,3-dihydro-imidazol-2-on | C23H27N303 | 393,49 | 394 |
| 112 | 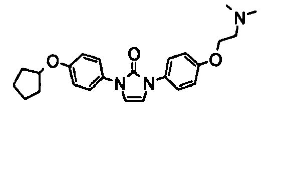 | 1-(4-Cyclopentyloxy-phenyl)-3-[4-(2-dimethylamino-ethoxy)-phenyl]-1,3-dihydro-imidazol-2-on | C24H29N303 | 407,52 | 408 |
| 113 | 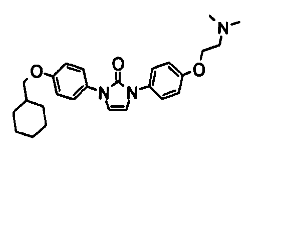 | 1-(4-Cyclohexylmethoxy-phenyl)-3-[4-(2-dimethylamino-ethoxy)-phenyl]-1,3-dihydro-imidazol-2-on | C26H33N303 | 435,57 | 436 |
| 114 | 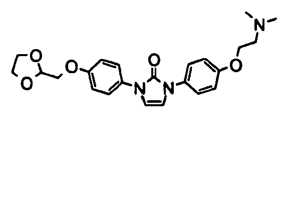 | 1-[4-(2-Dimethylamino-ethoxy)-phenyl]-3-[4-([1,3]dioxolan-2-ylmethoxy)-phenyl]-1,3-dihydro-imidazol-2-on | C23H27N305 | 425,49 | 426 |
| 115 | 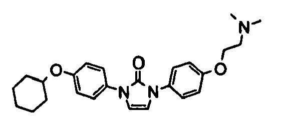 | 1-(4-Cyclohexyloxy-phenyl)-3-[4-(2-dimethylamino-ethoxy)-phenyl]-1,3-dihydro-imidazol-2-on | C25H31N303 | 421,54 | 422 |
| 116 | 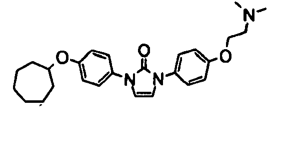 | 1-(4-Cycloheptyloxy-phenyl)-3-[4-(2-dimethylamino-ethoxy)-phenyl]-1,3-dihydro-imidazol-2-on | C26H33N303 | 435,57 | 436 |
| 117 | 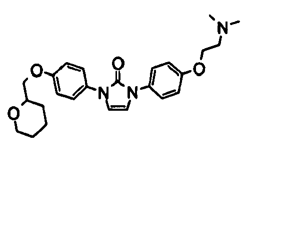 | 1-[4-(2-Dimethylamino-ethoxy)-phenyl]-3-[4-(tetrahydro-pyran-2-ylmethoxy)-phenyl]-1,3-dihydro-imidazol-2-on | C25H31N304 | 437,54 | 438 |
| 118 | 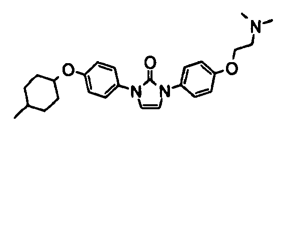 | 1-[4-(2-Dimethylamino-ethoxy)-phenyl]-3-[4-(4-methyl-cyclohexyloxy)-phenyl]-1,3-dihydro-imidazol-2-on | C26H33N303 | 435,57 | 436 |
| 119 | 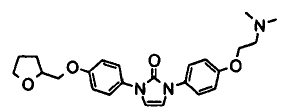 | 1-[4-(2-Dimethylamino-ethoxy)-phenyl]-3-[4-(tetrahydro-furan-2-ylmethoxy)-phenyl]-1,3-dihydro-imidazol-2-on | C24H29N304 | 423,52 | 424 |
| 122 | 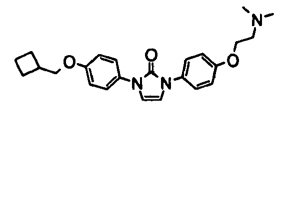 | 1-(4-Cyclobutylmethoxy-phenyl)-3-[4-(2-dimethylamino-ethoxy)-phenyl]-1,3-dihydro-imidazol-2-on | C24H29N303 | 407,52 | 408 |
| 125 | 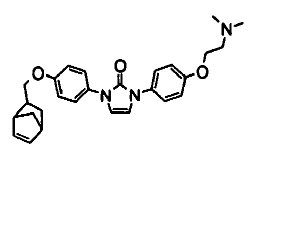 | 1-[4-(Bicyclo[2.2.1]hept-5-en-2-ylmethoxy)-phenyl]-3-[4-(2-dimethylamino-ethoxy)-phenyl]-1,3-dihydro-imidazol-2-on | C27H31N303 | 445,57 | 446 |
| 127 | 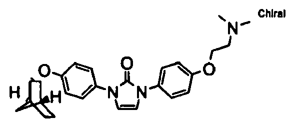 | 1-[4-((1S,2R,4R)-Bicyclo[2.2.1]hept-2-yloxy)-phenyl]-3-[4-(2-dimethylamino-ethoxy)-phenyl]-1,3-dihydro-imidazol-2-on | C26H31N303 | 433,56 | 434 |

### Beispiel 131

### 1-[4-(2-Dimethylamino-ethoxy)-phenyl]-3-[4-(2-nitro-phenoxy)-phenyl]-1,3-dihydroimidazol- 2-on

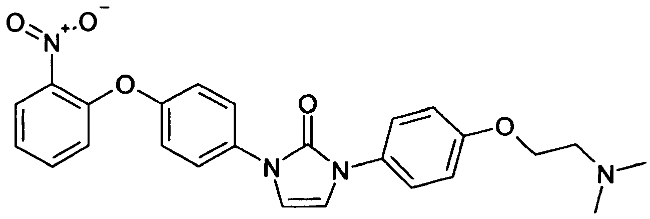

Zu einer Mischung von 1-[4-(2-Dimethylamino-ethoxy)-phenyl]-3-(4-hydroxy-phenyl)-1,3-dihydro-imidazol-2-on (0,34 g), Aliquat 336 (0,04 g), Kaliumhydroxid (0,077g) und Toluol (10 mL) wurde 2-Nitrofluorbenzol (0,14 g) gegeben. Die Mischung wurde für 3 Stunden zum Rückfluss erhitzt. Nach dem Abkühlen wurde die Reaktionslösung mit Wasser gewaschen, getrocknet und eingeengt. Der Rückstand wurde durch präparative HPLC gereinigt. Man erhielt so das Produkt mit dem Molekulargewicht 460,49 (C₂₅H₂₄N₄O₅); MS (ESI): 461 ([M+H]⁺) als Hydrotrifluoracetat.

### Beispiel 132

### 1-[4-(2-Dimethylamino-ethoxy)-phenyl]-3-(4-o-tolyloxy-phenyl)-1,3-dihydro-imidazol-2-on

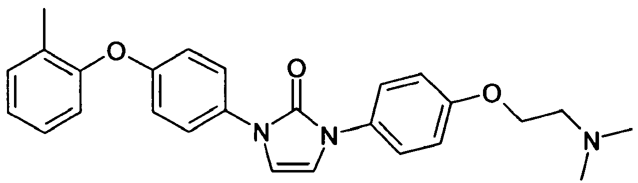

Zu einer Lösung von 4-o-Tolyloxy-phenylamin (50 mg) in Dimethylformamid (2 mL) wurde bei 0 °C Carbonyldiimidazol (40 mg) gegeben. Nach 30 Minuten wurde (2,2-Dimethoxy-ethyl)-[4-(2-dimethylamino-ethoxy)-phenyl]-amin (67 mg) zugesetzt und die Mischung für 2 Stunden auf 80 °C erwärmt. Nach dem Abkühlen auf Raumtemperatur wurde Trifluoressigsäure (0,5 mL) zugesetzt und 72 Stunden stehen gelassen. Die Reaktionslösung wurde mit Ethylacetat verdünnt und mit gesättigter Natriumhydrogencarbonatlösung gewaschen. Die organische Phase wurde getrocknet und eingeengt. Der Rückstand wurde durch präparative HPLC gereinigt. Man erhielt so das Produkt mit dem Molekulargewicht 429,52 (C₂₆H₂₇N₃O₃); MS (ESI): 430 ([M+H]⁺) als Hydrotrifluoracetat.

### (2,2-Dimethoxy-ethyl)-[4-(2-dimethylamino-ethoxy)-phenyl]-amin

Eine Lösung von 4-(2,2-Dimethoxy-ethylamino)-phenol (4,3 g) in Dimethylformamid (25 mL) wurde mit Natriumhydrid (1,57 g) versetzt. Nach 30 Minuten wurde 2-Dimethylaminoethylchlorid (Hydrochlorid, 3,14 g) zugesetzt. Nach 12 Stunden wurde die Reaktionslösung mit Ethylacetat verdünnt und mit Wasser gewaschen. Die organische Phase wurde getrocknet und eingeengt. Der Rückstand wurde durch MPLC (Eluent: Dichlormethan/Methanol/Ammoniaklösung 95 : 4,9 : 0,1) gereinigt. Man erhielt so das Produkt mit dem Molekulargewicht 268.36 (C₁₄H₂₄N₂O₃); MS (ESI): 269 ([M+H]⁺).

### 4-(2,2-Dimethoxy-ethylamino)-phenol

Eine Lösung von (4-Benzyloxy-phenyl)-(2,2-dimethoxy-ethyl)-amin (3,5 g) in Ethanol (50 mL) wurde unter Stickstoff mit Palladium(II)hydroxid (20% auf Kohle, 0,5 g) versetzt. Die Stickstoffatmosphäre wurde durch Wasserstoff ausgetauscht und die Mischung für 3 Stunden geschüttelt. Der Katalysator wurde abfiltriert und das Filtrat eingeengt. Das Rohprodukt wurde ohne Reinigung weiter umgesetzt.

### Beispiele 133-166

Weitere Beispiele, die wie im Beispiel 132 beschrieben unter Verwendung des entsprechenden Anilins hergestellt wurden, sind in der Tabelle 4 zusammengefasst.

**Tabelle 4**

| Bsp. No. | Name | Struktur | Summenformel | Molekula r-gewicht | [M+H]⁺ ESI |
|---|---|---|---|---|---|
| 133 | 1-[4-(2-Chlor-phenoxy)-phenyl]-3-[4-(2-dimethylamino-ethoxy)-phenyl]-1,3-dihydro-imidazol-2-on | 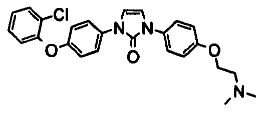 | C25H24ClN3O3 | 449,94 | 450 |
| 134 | 1-[4-(2-Dimethylamino-ethoxy)-phenyl]-3-[4-(2-methoxy-phenoxy) phenyl]-1,3-dihydro-imidazol-2-on | 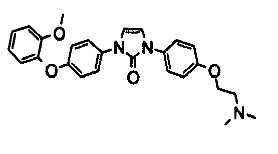 | C26H27N304 | 445,52 | 446 |
| 135 | 1-[4-(2-Dimethylamino-ethoxy)-phenyl]-3-(4-m-tolyloxy-phenyl)-1,3-dihydro-imidazol-2-on | 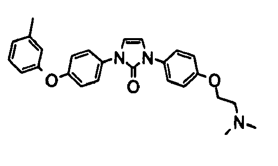 | C26H27N303 | 429,52 | 430 |
| 136 | 1-[4-(3-Chlor-phenoxy)-phenyl]-3-[4-(2-dimethylamino-ethoxy)-phenyl]-1,3-dihydro-imidazol-2-on | 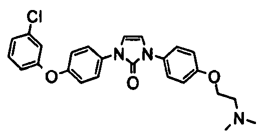 | C25H24ClN3O3 | 449,94 | 450 |
| 137 | 1-[4-(2-Dimethylamino-ethoxy)-phenyl]-3-[4-(3-methoxy-phenoxy)-phenyl]-1,3-dihydro-imidazol-2-on | 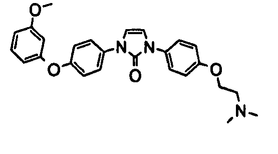 | C26H27N304 | 445,52 | 446 |
| 138 | 1-[4-(2-Dimethylamino-ethoxy)-phenyl]-3-[4-(pyridin-3-yloxy)-phenyl]-1,3-dihydro-imidazol-2-on | 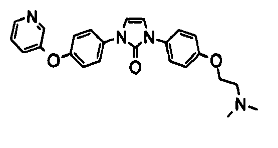 | C24H24N403 | 416,48 | 417 |
| 141 | 1-[4-(2-Dimethylamino-ethoxy)-phenyl]-3-(2-methoxy-4-phenylamino-phenyl)-1,3-dihydro-imidazol-2-on | 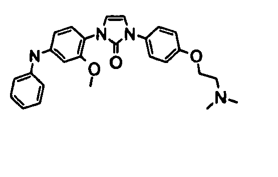 | C26H28N403 | 444,54 | 445 |
| 142 | 1-(4-Benzyloxy-phenyl)-3-[4-(2-dimethylamino-ethoxy)-phenyl]-1,3-dihydro-imidazol-2-on | 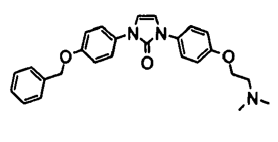 | C26H27N303 | 429,52 | 430 |
| 143 | 1-(4-Benzyl-phenyl)-3-[4-(2-dimethylamino-ethoxy)-phenyl]-1,3-dihydro-imidazol-2-on | 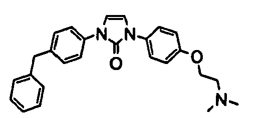 | C26H27N302 | 413,52 | 414 |
| 144 | 1-[4-(2-Dimethylamino-ethoxy)-phenyl]-3-(4-pyridin-4-ylmethyl-phenyl)-1,3-dihydro-imidazol-2-on | 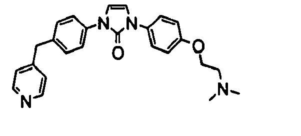 | C25H26N402 | 414,51 | 415 |
| 145 | 1-[4-(2-Dimethylamino-ethoxy)-phenyl]-3-(4-p-tolyloxy-phenyl)-1,3-dihydro-imidazol-2-on | 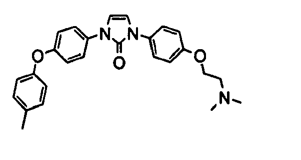 | C26H27N303 | 429,52 | 430 |
| 146 | 1-[4-(2-Dimethylamino-ethoxy)-phenyl]-3-(4-phenylsulfanyl-phenyl)-1,3-dihydro-imidazol-2-on | 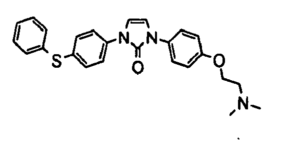 | C25H25N3O2S | 431,56 | 432 |
| 147 | 1-[4-(2-Dimethylamino-ethoxy)-phenyl]-3-[4-(3-trifluormethyl-phenoxy)-phenyl]-1,3-dihydro-imidazol-2-on | 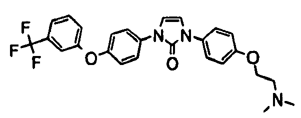 | C26H24F3N303 | 483,49 | 484 |
| 150 | 1-[3-Chlor-4-(pyrimidin-2-yloxy)-phenyl]-3-[4-(2-dimethylamino-ethoxy)-phenyl]-1,3-dihydro-imidazol-2-on | 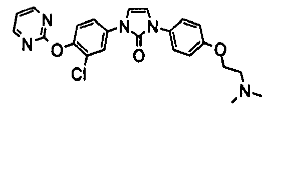 | C23H22ClN5O3 | 451,92 | 452 |
| 152 | 1-[4-(2-Dimethylamino-ethoxy)-phenyl]-3-[4-(piperidine-1-carbonyl)-phenyl]-1,3-dihydro-imidazol-2-on | 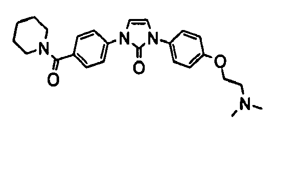 | C25H30N403 | 434,54 | 435 |
| 154 | 1-[4-(2-Dimethylamino-ethoxy)-phenyl]-3-[4-(4-fluoro-phenoxy)-phenyl]-1,3-dihydro-imidazol-2-on | 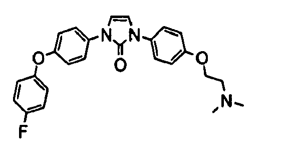 | C25H24FN303 | 433,49 | 434 |
| 155 | 1-[4-(2-Dimethylamino-ethoxy)-phenyl]-3-[4-(pyrimidin-2-yloxy)-phenyl]-1,3-dihydro-imidazol-2-on | 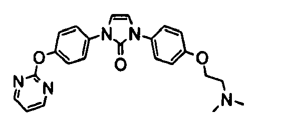 | C23H23N503 | 417,47 | 418 |
| 159 | 1-[4-(2-Dimethylamino-ethoxy)-phenyl]-3-[4-(thiophen-2-ylsulfanyl)-phenyl]-1,3-dihydro-imidazol-2-on | 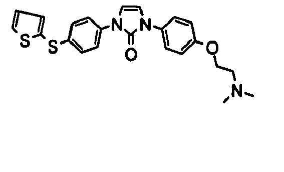 | C23H23N3O2S2 | 437,59 | 438 |
| 161 | 1-[4-(2-Dimethylamino-ethoxy)-phenyl]-3-[4-(2-fluor-phenoxy)-phenyl]-1,3-dihydro-imidazol-2-on | 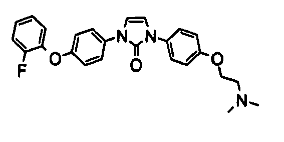 | C25H24FN303 | 433,49 | 434 |
| 162 | 1-(3-Cyclopentyloxy-4-methoxy-phenyl)-3-[4-(2-dimethylamino-ethoxy)-phenyl]-1,3-dihydro-imidazol-2-on | 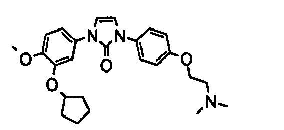 | C25H31N304 | 437,54 | 438 |

### Beispiel 164

### 1-(4-Cyclopentyloxy-phenyl)-3-{4-[(2-hydroxy-ethyl)-methyl-amino]-phenyl}-1,3-dihydro-imidazol-2-on

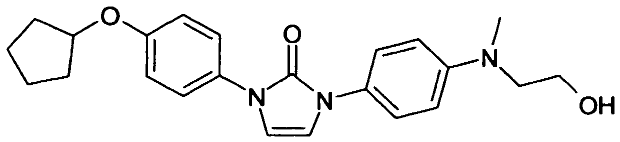

Eine Lösung von 1-(4-Cyclopentyloxy-phenyl)-1-(2,2-dimethoxy-ethyl)-3-{4-[(2-hydroxy-ethyl)-methyl-amino]- phenyl}-harnstoff (0,4 g) in Dimethylformamid (6 mL) wurde mit Trifluoressigsäure (2 mL) versetzt und für 48 Stunden stehen gelassen. Die Mischung wurde mit Wasser verdünnt und mit Ethylacetat extrahiert. Die organische Phase wurde mit Wasser und Natriumcarbonatlösung gewaschen, getrocknet und eingeengt. Man erhielt so das Produkt mit dem Molekulargewicht 393,49 (C₂₃H₂₇N₃O₃); MS (ESI): 394 ([M+H]⁺).

### 1-(4-Cyclopentyloxy-phenyl)-1-(2,2-dimethoxy-ethyl)-3-{4-[(2-hydroxy-ethyl)-methylamino]- phenyl}-harnstoff

Nach der in Beispiel 4 gegebenen Vorschrift wurde 2-[(4-Amino-phenyl)-methylamino]-ethanol mit Carbonyldiimidazol und (4-Cyclopentyloxy-phenyl)-(2,2-dimethoxyethyl)-amin umgesetzt. Das Rohprodukt wurde durch Chromatographie an Kieselgel (Eluent: Ethylacetat) gereinigt. Man erhielt so das Produkt mit dem Molekulargewicht 457,57 (C₂₅H₃₅N₃O₅); MS (ESI): 458 ([M+H]⁺).

### 2-[(4-Amino-phenyl)-methyl-amino]-ethanol

Eine Lösung von 2-[Methyl-(4-nitro-phenyl)-amino]-ethanol (6 g) in Methanol (100 mL) wurde unter Stickstoff mit Palladium (10% auf Kohle, 1 g) versetzt. Die Stickstoffatmosphäre wurde durch Wasserstoff ausgetauscht und die Mischung für 4 Stunden geschüttelt. Der Katalysator wurde abfiltriert und das Filtrat eingeengt. Man erhielt so das Produkt mit dem Molekulargewicht 166,22 (C₉H₁₄N₂O); MS (ESI): 167 ([M+H]⁺).

### 2-[Methyl-(4-nitro-phenyl)-amino]-ethanol

Eine Mischung aus 4-Fluornitrobenzol (20 g) und 2-Methylaminoethanol (56 mL) wurde für 12 Stunden stehen gelassen und dann mit Ethylacetat verdünnt. Es wurde mit Wasser gewaschen. Die organische Phase wurde getrocknet und eingeengt. Man erhielt so das Produkt mit dem Molekulargewicht 196,21 (C₉H₁₂N₂O₃); MS (ESI): 197 ([M+H]⁺).

### Beispiel 165

### 1-(4-Cyclopentyloxy-phenyl)-3-{4-[(2-imidazol-1-yl-ethyl)-methyl-amino]-phenyl}-1,3-dihydro-imidazol-2-on

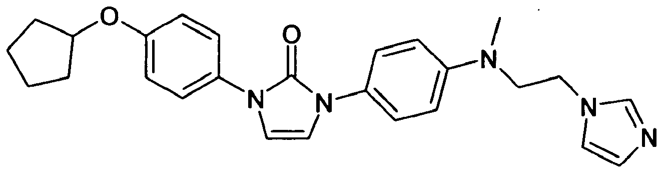

Eine Lösung von 1-(4-Cyclopentyloxy-phenyl)-3-{4-[(2-hydroxy-ethyl)-methyl-amino]-phenyl}-1,3-dihydro-imidazol-2-on (0,20 g) in Dichlormethan (5 mL) wurde bei 0 °C mit Triethylamin (0,13 g) und Methansulfonsäurechlorid (0,1 mL) versetzt. Nach zwei Stunden wurden flüchtige Anteile entfernt und der Rückstand in Propionitril (5 mL) aufgenommen. Es wurde Imidazol (0,25 g) zugesetzt und für 6 Stunden auf 90 °C erwärmt. Flüchtige Anteile wurden entfernt und der Rückstand durch präparative HPLC gereinigt. Man erhielt so das Produkt mit dem Molekulargewicht 443,55 (C₂₆H₂₉N₅O₂); MS (ESI): 444 ([M+H]⁺) als Hydrotrifluoracetat.

### Beispiel 166

### 1-(4-Cyclopentyloxy-phenyl)-3-(4-{methyl-[2-(2-methyl-imidazol-1-yl)-ethyl]-amino}-phenyl)-1,3-dihydro-imidazol-2-on

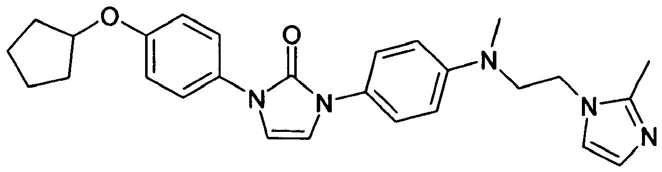

Die Verbindung wurde wie im Beispiel 165 beschrieben nur unter Verwendung von 2-Methylimidazol hergestellt. Man erhielt so das Produkt mit dem Molekulargewicht 457,58 (C₂₇H₃₁N₅O₂); MS (ESI): 458 ([M+H]⁺) als Hydrotrifluoracetat.

### Beispiel 167

### 1-(4-Cyctopentyloxy-phenyl)-3-{4-[methylt-(2-methylamino-ethyl)-amino]-phenyl}-1,3-dihydro-imidazol-2-on

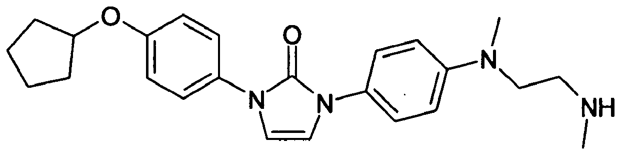

Die Verbindung wurde wie im Beispiel 165 beschrieben nur unter Verwendung von Methylamin (1 M in THF) hergestellt. Man erhielt so das Produkt mit dem Molekulargewicht 406,53 (C₂₄H₃₀N₄O₂); MS (ESI): 407 ([M+H]⁺) als Hydrotrifluoracetat.

### Beispiel 168

### 1-(4-Cyclopentyloxy-phenyl)-3-{4-[methyl-(2-piperidin-1-yl-ethyl)-amino]-phenyl}-1,3-dihydro-imidazol-2-on

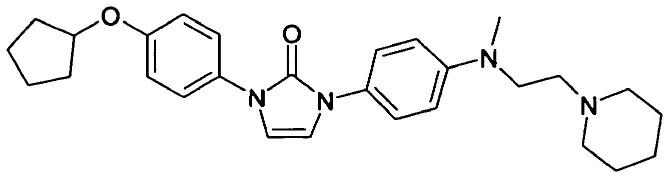

Die Verbindung wurde wie im Beispiel 165 beschrieben nur unter Verwendung von Piperidin hergestellt. Man erhielt so das Produkt mit dem Molekulargewicht 460,62 (C₂₈H₃₆N₄O₂); MS (ESI): 461 ([M+H]⁺) als Hydrotrifluoracetat.

### Beispiel 170

### N-[2-({4-[3-(4-Cyclopentyloxy-phenyl)-2-oxo-2,3-dihydro-imidazol-1-yl]-phenyl}-methylamino)-ethyl]-N-methyl-acetamid

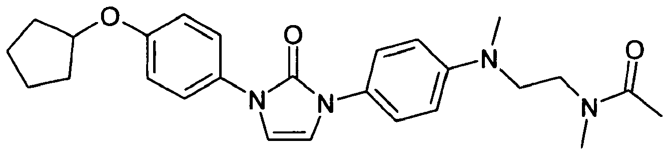

Eine Lösung von 1-(4-Cyclopentyloxy-phenyl)-3-{4-[methyl-(2-methylamino-ethyl)-amino]-phenyl}-1,3-dihydro-imidazol-2-on (50 mg) in Dichlormethan (2 mL) wurde mit Triethylamin (25 mg) und Acetylchlorid (15 mg) versetzt. Nach 2 Stunden wurden flüchtige Anteile entfernt und der Rückstand durch präparative HPLC gereinigt. Man erhielt so das Produkt mit dem Molekulargewicht 448,57 (C₂₆H₃₂N₄O₃); MS (ESI): 449 ([M+H]⁺) als Hydrotrifluoracetat.

### Beispiel 174

### 4-[4-(2-Dimethylamino-ethoxy)-phenyl]-2-(4-phenoxy-phenyl)-2,4-dihydro-[1,2,4]triazol-3-on

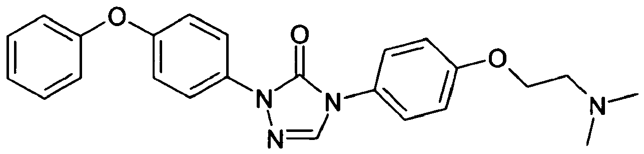

Zu einer Lösung von Kaliumhydroxid (15 mg) in Methanol (20 mL) wurde 1-Formyl-2-(4-phenoxy-phenyl)-4-[4-(2-dimethylamino-ethoxy)-phenyl]-semicarbazid (60 mg) gegeben. Nach 4 Stunden bei Raumtemperatur wurde die Mischung für 24 Stunden auf 40°C erhitzt. Nach dem Abkühlen wurde die Reaktionslösung mit Wasser verdünnt und mit Ethylacetat extrahiert. Die organische Phase wurde getrocknet und eingeengt. Man erhielt so das Produkt mit dem Molekulargewicht 416,48 (C₂₄H₂₄N₄O₃); MS (ESI): 417 ([M+H]⁺) .

1 -Formyl-2-(4-phenoxy-phenyl)-4-[4-(2-dimethylamino-ethoxy)-phenyl]-semicarbazid Zu einer Lösung von 1-Benzyliden-2-(4-phenoxy-phenyl)-4-[4-(2-dimethylaminoethoxy)-phenyl]-semicarbazid (600 mg) in Tetrahydrofuran / Ethanol (1:1, 30 mL) wurden Palladium(II)hydroxid (60 mg) und Ameisensäure (4.6 g) zugesetzt. Die Mischung wurde für fünf Stunden zum Rückfluss erhitzt und dann filtriert. Das Filtrat wurde eingeengt und der Rückstand mittels HPLC gereinigt. Man erhielt so das Produkt mit dem Molekulargewicht 434,50 (C₂₄H₂₆N₄O₄); MS (ESI): 435 ([M+H]⁺).

### 1-Benzyliden-2-(4-phenoxy-phenyl)-4-[4-(2-dimethylamino-ethoxy)-phenyl]-semicarbazid

Zu einer Lösung von N-Benzyliden-N'-(4-phenoxy-phenyl)-hydrazin (50 mg) in Toluol (1 mL) wurde bei 0 °C Phosgen (20 % in Toluol; 0,091 mL) getropft. Nach 24 Stunden bei Raumtemperatur wurde 4-(2-Dimethylamino-ethoxy)-anilin (31 mg) zugesetzt. Nach zwei Stunden wurden die ausgefallenen Kristalle abgesaugt. Man erhielt so das Produkt mit dem Molekulargewicht 494,60 (C₃₀H₃₀N₄O₃); MS (ESI): 495 ([M+H]⁺).

### N-Benzyliden-N'-(4-phenoxy-phenyl)-hydrazin

Eine Suspension von (4-Phenoxy-phenyl)-hydrazin (J. Org. Chem. 1956, 395; 1,5 g) in Methanol (10 mL) wurde mit Benzaldehyd (0,76 mL) versetzt und zum Rückfluss erhitzt. Die beim Abkühlen ausgefallenen Kristalle wurden abgesaugt. Man erhielt so das Produkt mit dem Molekulargewicht 288,35 (C₁₉H₁₆N₂O); MS (ESI): 289 ([M+H]⁺).

### Beispiel 175

### 1-(4-Cyclopentyloxy-phenyl)-3-[4-(2-methylamino-ethoxy)-phenyl]-1,3-dihydroimidazol-2-on

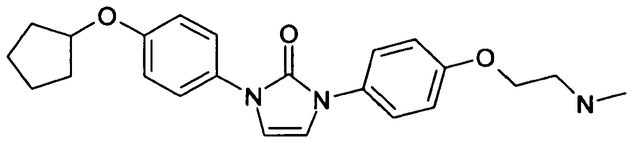

1-[4-(2-Brom-ethoxy)-phenyl]-3-(4-cyclopentyloxy-phenyl)-1,3-dihydro-imidazol-2-on wurde wie im Beispiel 6 beschrieben mit Methylamin umgesetzt. Man erhielt so das Produkt mit dem Molekulargewicht 393,49 (C₂₃H₂₇N₃O₃); MS (ESI): 394 ([M+H]⁺) .

1-[4-(2-Brom-ethoxy)-phenyl]-3-(4-cyclopentyloxy-phenyl)-1,3-dihydro-imidazol-2-on 1-(4-Cyclopentyloxy-phenyl)-3-(4-hydroxy-phenyl)-1,3-dihydro-imidazol-2-on wurde wie im Beispiel 5 beschrieben mit 1,2-Dibromethan umgesetzt. Man erhielt so das Produkt mit dem Molekulargewicht 443,34 (C₂₂H₂₃BrN₂O₃); MS (ESI): 443 ([M+H]⁺) .

### 1-(4-Cyclopentyloxy-phenyl)-3-(4-hydroxy-phenyl)-1,3-dihydro-imidazol-2-on

3-(4-Benzyloxy-phenyl)-1-(4-cyclopentyloxy-phenyl)-1-(2,2-dimethoxy-ethyl)-harnstoff wurde wie im Beispiel 92 beschrieben zunächst mit Wasserstoff und dann mit TFA umgesetzt. Man erhielt so das Produkt mit dem Molekulargewicht 336,39 (C₂₀H₂₀N2O₃); MS (ESI): 337 ([M+H]⁺) .

3-(4-Benzyloxy-phenyl)-1-(4-cyclopentyloxy-phenyl)-1-(2,2-dimethoxy-ethyl)-harnstoff (4-Cyclopentyloxy-phenyl)-(2,2-dimethoxy-ethyl)-amin wurde wie im Beispiel 5 beschrieben mit Carbonyldiimidazol und 4-Benzyloxyanilin umgesetzt. Man erhielt so das Produkt mit dem Molekulargewicht 490,60 (C₂₉H₃₄N₂O₅); MS (ESI): 491 ([M+H]⁺) .

### Beispiel 176

### N-(2-{4-[3-(4-Cyclopentyloxy-phenyl)-2-oxo-2,3-dihydro-imidazol-1-yl]-phenoxy}-ethyl)-N-methyl-acetamid

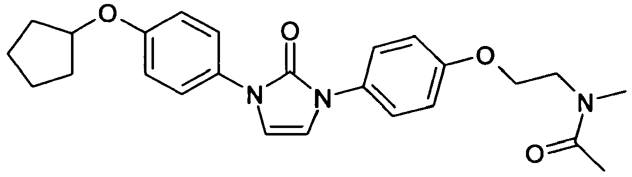

1-(4-Cyclopentyloxy-phenyl)-3-[4-(2-methylamino-ethoxy)-phenyl]-1,3-dihydroimidazol-2-on wurde wie im Beispiel 170 beschrieben mit Acetylchlorid und Triethylamin umgesetzt. Man erhielt so das Produkt mit dem Molekulargewicht 435,53 (C₂₅H₂₉N₃O₄); MS (ESI): 436 ([M+H]⁺) .

### Beispiel 177

### 1-(4-Cyclopentyloxy-phenyl)-3-[4-(2-[1,2,4]triazol-4-yl-ethoxy)-phenyl]-1,3-dihydroimidazol-2-on

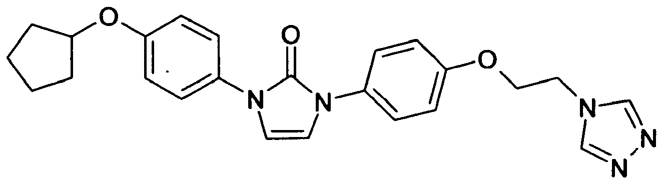

1-[4-(2-Brom-ethoxy)-phenyl]-3-(4-cyclopentyloxy-phenyl)-1,3-dihydro-imidazol-2-on wurde wie im Beispiel 5 beschrieben mit 1,2,4-Triazol umgesetzt. Man erhielt so das Produkt mit dem Molekulargewicht 431,50 (C₂₄H₂₅N₅O₃); MS (ESI): 432 ([M+H]⁺) .

### Beispiele 179-216

Weitere Beispiele wurden durch Umsetzung von 1-[4-(2-Brom-ethoxy)-phenyl]-3-(4-cyclopentyloxy-phenyl)-1,3-dihydro-imidazol-2-on mit den entsprechenden Aminen erhalten. Die Ergebnisse sind in der Tabelle 5 zusammengefasst.

**Tabelle 5**

| Bsp. No. | Name | Struktur | Summen-formel | Molekular-gewicht | [M+H]+ ESI-MS |
|---|---|---|---|---|---|
| 181 | 1-{4-[2-(4-Acetyl-piperazin-1-yl)-ethoxy]-phenyl}-3-(4-cyclopentyloxy phenyl)-1,3-dihydro-imidazol-2-on | 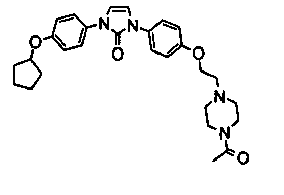 | C28H34N404 | 490,61 | 491 |
| 182 | 1-(4-Cyclopentyloxy-phenyl)-3-{4-[2 (4-hydroxy-piperidin-1-yl)-ethoxy]-phenyl}-1,3-dihydro-imidazol-2-on | 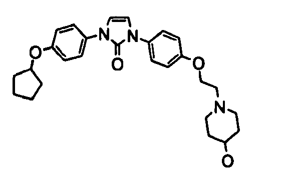 | C27H33N304 | 463,58 | 464 |
| 183 | 1-(4-Cyclopentyloxy-phenyl)-3-{4-[2 (4-methyl-piperid in-1-yl)-ethoxy]-phenyl}-1,3-dihydro-imidazol-2-on | 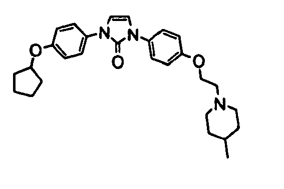 | C28H35N303 | 461,61 | 462 |
| 185 | 1-[4-(2-Cyclohexylamino-ethoxy)-phenyl]-3-(4-cyclopentyloxy-phenyl) 1,3-dihydro-imidazol-2-on | 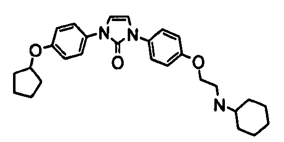 | C28H35N303 | 461,61 | 462 |
| 186 | 1-(4-Cyclopentyloxy-phenyl)-3-[4-(2-isopropylamino-ethoxy)-phenyl]-1,3-dihydro-imidazol-2-on | 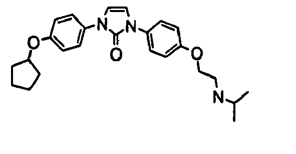 | C25H31N303 | 421,54 | 422 |
| 187 | 1-(4-Cyclopentyloxy-phenyl)-3-{4-[2-(4-ethyl-piperazin-1-yl)-ethoxy]-phenyl}-1,3-dihydro-imidazol-2-on | 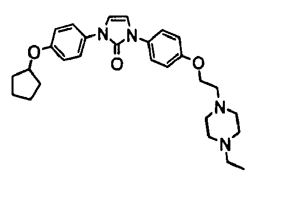 | C28H36N403 | 476,62 | 477 |
| 188 | 1-(4-Cyclopentyloxy-phenyl)-3-{4-[2-(2,5-dimethyl-pyrrolidin-1-yl)-ethoxy]-phenyl}-1,3-dihydro-imidazol-2-on | 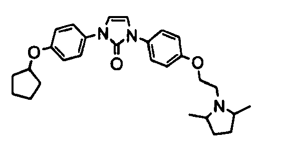 | C28H35N303 | 461,61 | 462 |
| 189 | 1-(4-Cyclopentyloxy-phenyl)-3-{4-[2-(isopropyl-methyl-amino)-ethoxy]-phenyl}-1,3-dihydro-imidazol-2-on | 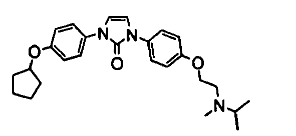 | C26H33N303 | 435,57 | 436 |
| 193 | N-[1-(2-{4-[3-(4-Cyclopentyloxy-phenyl)-2-oxo-2,3-dihydro-imidazol-1-yl]-phenoxy}-ethyl)-pyrrolidin-3-yl]-acetamid | 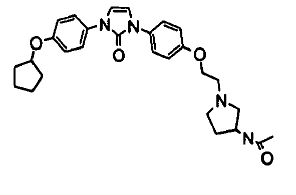 | C28H34N404 | 490,61 | 491 |
| 195 | 1-(4-Cyclopentyloxy-phenyl)-3-{4-[2-(3-hydroxy-pyrrolidin-1-yl)-ethoxy]-phenyl}-1,3-dihydro-imidazol-2-on | 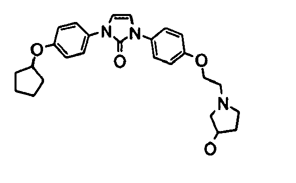 | C26H31N304 | 449,55 | 450 |
| 196 | 1-(4-Cyclopentyloxy-phenyl)-3-{4-[2-(2-methyl-pyrrolidin-1-yl)-ethoxy]-phenyl}-1,3-dihydro-imidazol-2-on | 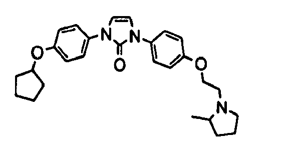 | C27H33N303 | 447,58 | 448 |
| 197 | (S)-1-(2-{4-[3-(4-Cyclopentyloxy-phenyl)-2-oxo-2,3-dihydro-imidazol-1-yl]-phenoxy}-ethyl)-pyrrolidin-2-carbonsäuredimethylamid | 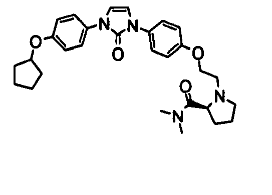 | C29H36N404 | 504,63 | 505 |
| 199 | 1-(4-Cyclopentyloxy-phenyl)-3-{4-[2-(2-methyl-imidazol-1-yl)-ethoxy]-phenyl}-1,3-dihydro-imidazol-2-on | 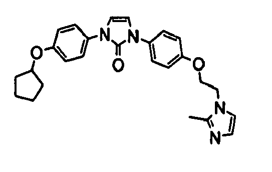 | C26H28N403 | 444,54 | 445 |
| 200 | 1-(4-Cyclopentyloxy-phenyl)-3-{4-[2-(4-methyl-imidazol-1-yl)-ethoxy]-phenyt}-1,3-dihydro-imidazol-2-on | 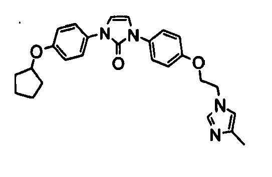 | C26H28N403 | 444,54 | 445 |
| 201 | 1-(4-Cyclopentyloxy-phenyl)-3-{4-[2-(2-ethyl-imidazol-1-yl)-ethoxy]-phenyl}-1,3-dihydro-imidazol-2-on | 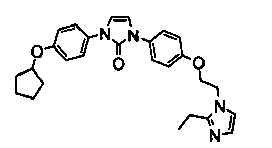 | C27H30N403 | 458,57 | 459 |
| 202 | 1-(4-Cyclopentyloxy-phenyl)-3-{4-[2-(2,4-dimethyl-imidazol-1-yl)-ethoxy]-phenyl}-1,3-dihydro-imidazol-2-on | 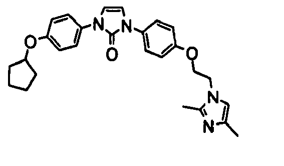 | C27H30N403 | 458,57 | 459 |
| 204 | 1-(4-Cyclopentyloxy-phenyl)-3-{4-[2-(2-ethyl-4-methyl-imidazol-1-yl)-ethoxy]-phenyl}-1,3-dihydro-imidazol-2-on | 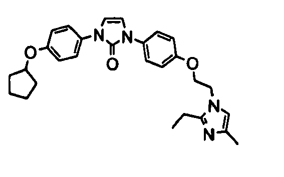 | C28H32N403 | 472,59 | 473 |
| 205 | 1-(4-Cyclopentyloxy-phenyl)-3-{4-[2-(2-isopropyl-imidazol-1-yl)-ethoxy]-phenyl}-1,3-dihydro-imidazol-2-on | 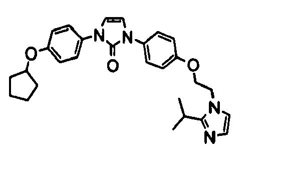 | C28H32N403 | 472,59 | 473 |
| 206 | 1-(4-Cyclopentyloxy-phenyl)-3-{4-[2-(2,4,5-trimethyl-imidazol-1-yl)-ethoxy]-phenyl}-1,3-dihydro-imidazol-2-on | 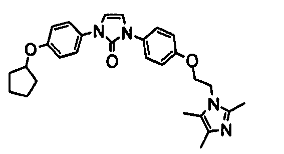 | C28H32N403 | 472,59 | 473 |
| 214 | 1-(4-Cyclopentyloxy-phenyl)-3-{4-[2-(2-methyl-4,5-dihydro-imidazol-1-yl)-ethoxy]-phenyl}-1,3-dihydro-imidazol-2-on | 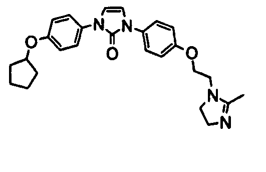 | C26H30N403 | 446,55 | 447 |
| 215 | 1-(4-Cyclopentyloxy-phenyl)-3-{4-[2-(4,4-dimethyl-4,5-dihydro-imidazol-1-yl)-ethoxy]-phenyl}-1,3-dihydro-imidazol-2-on | 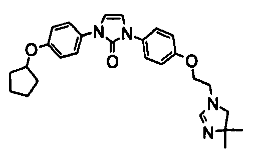 | C27H32N403 | 460,58 | 461 |

### Beispiel 216

### 1-(4-Amino-phenyl)-3-(4-phenoxy-phenyl)-1,3-dihydro-imidazol-2-on

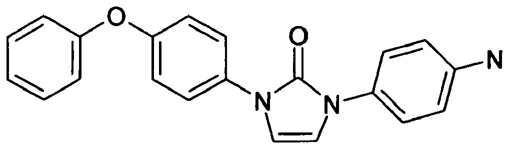

Eine Lösung von N-{4-[2-Oxo-3-(4-phenoxy-phenyl)-2,3-dihydro-imidazol-1-yl]-phenyl}-acetamid (4,0 g) in Ethanol (100 mL) wurde mit Salzsäure (20 %ig, 30 mL) für 16 Stunden zum Rückfluss erhitzt. Nach dem Abkühlen wurde Ammoniak-Lösung (10 %ig) bis zur basischen Reaktion zugesetzt. Der ausgefallene Feststoff wurde abfiltriert und im Vakuumofen bei 40°C getrocknet. Man erhielt so das Produkt mit dem Molekulargewicht 343,39 (C₂₁H₁₇N₃O₂); MS (ESI): 344 ([M+H]⁺).

### N-{4-[2-Oxo-3-(4-phenoxy-phenyl)-2,3-dihydro-imidazol-1-yl]-phenyl}-acetamid

Wie im Beispiel 92 beschrieben wurde 4-Acetaminoanilin mit Carbonyldiimidazol und (2,2-Dimethoxy-ethyl)-(4-phenoxy-phenyl)-amin umgesetzt gefolgt von einer Behandlung des Rohprodukts mit Trifluoressigsäure bei 80 °C. Bei der Zugabe von Ethylacetat / Heptan 1:1 präzipitierte das Produkt. Der ausgefallene Feststoff wurde abfiltriert und im Vakuumofen bei 40 °C getrocknet. Man erhielt so das Produkt mit dem Molekulargewicht 385,43 (C₂₃H₁₉N₃O₃); MS (ESI): 386 ([M+H]⁺).

## Patentansprüche

1. Verbindungen der Formel I, 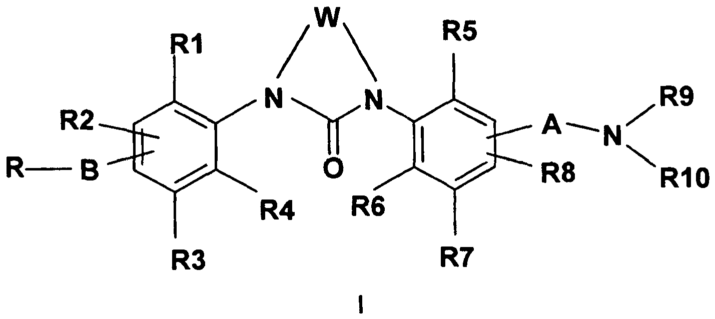 worin bedeuten
R (C₃-C₈)-Cycloalkyl; 5-6 gliedriger Ring, der ein oder zwei Heteroatome aus der Gruppe N, O und S enthalten kann und der 5-6 gliedrige Ring weitere Substituenten ausgewählt aus F, Cl, Br, NO₂, CF₃, OCF₃, CN, (C₁-C₆)-Alkyl, O-(C₁-C₆)-Alkyl tragen kann;
B O, S, CO, OCH₂, N(R23), CH₂;
R23 H, (C₁-C₆)-Alkyl;
R1, R2, R3 , R4 unabhängig voneinander H, F, Cl, Br, CF₃, O-(C₁-C₆)-Alkyl, (C₁-C₆)-Alkyl;
W -CH=CH-, -N=CH-;
R5, R6, R7, R8 unabhängig voneinander H, F, Cl, Br, OH, CF₃, NO₂, CN, OCF₃, O-(C₁-C₆)-Alkyl;
A eine Kette -(C(R42)(R43))ₘ -, in der ein Glied ersetzt sein kann durch ein Element aus der Gruppe O, N(R44);
m 3, 4;
R42, R43, R44 unabhängig voneinander H, (C₁-C₆)-Alkyl, Aryl;
R9, R10 unabhängig voneinander H, (C₁-C₈)-Alkyl, -(CH₂)ₒ -R45, CO-(C₁-C₈)-Alkyl; oder R9 und R10 bilden zusammen mit dem Stickstoffatom, an das sie gebunden sind, einen 4 bis 10-gliedrigen mono-, bi- oder spiro-cyclischen Ring, welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, (C₁-C₆)-Alkyl, O-(C₁-C₈)-Alkyl, oxo, CO(R46), CON(R47)(R48), Hydroxy, N(R50)CO(C₁-C₆)-Alkyl oder N(R51)(R52);
R46, R47, R48, R50, R51, R52 unabhängig voneinander H, (C₁-C₆)-Alkyl;
o 0, 1, 2, 3, 4;
R45 OH, 5-10 gliedriger mono- oder bicyclischer Ring, der ein oder zwei Heteroatome aus der Gruppe N, O und S enthalten kann und der 5-10 gliedrige Ring weitere Substituenten ausgewählt aus F, Cl, Br, OH, CF₃, oxo, O-(C₁-C₆)-Alkyl, (C₁-C₆)-Alkyl, (C₀-C₂)-Alkylen-Aryl, O-(C₀-C₂)-Alkylen-Aryl oder N(R51)(R52) enthalten kann;
sowie deren physiologisch verträglichen Salze,
wobei die Alkyl-, Alkenyl- und Alkinylreste in den Substituenten R, R1, R2, R3, R4, R5, R6, R7, R8, R9. R10, R23, R42, R43, R44, R45, R46, R47, R48, R50, R51 und R52 sowohl geradkettig, verzweigt oder optional halogeniert sein können.

2. Verbindungen der Formel I gemäß dem Anspruch 1, worin
W -C=C- ist.

3. Arzneimittel enthaltend eine oder mehrere der Verbindungen gemäß den Ansprüchen 1 oder 2.

4. Arzneimittel enthaltend eine oder mehrere der Verbindungen gemäß den Ansprüchen 1 oder 2 und ein oder mehrere anorektische Wirkstoffe.

5. Verbindungen gemäß den Ansprüchen 1 oder 2 zur Anwendung als Medikament zur Prophylaxe oder Behandlung der Obesitas.

6. Verbindungen gemäß den Ansprüchen 1 oder 2 zur Anwendung als Medikament zur Prophylaxe oder Behandlung des Typ II Diabetes.

7. Verbindungen gemäß den Ansprüchen 1 bis 4 zur Anwendung als Medikament zur Behandlung von Störungen des Empfindens und anderer psychiatrischen Indikationen.

8. Verbindungen gemäß den Ansprüchen 1 oder 2 in Kombination mit mindestens einem weiteren anorektischen Wirkstoff zur Anwendung als Medikament zur Prophylaxe oder Behandlung der Obesitas.

9. Verbindungen gemäß den Ansprüchen 1 oder 2 in Kombination mit mindestens einem weiteren anorektischen Wirkstoff zur Anwendung als Medikament zur Prophylaxe oder Behandlung der des Typ II Diabetes.

10. Verbindungen gemäß den Ansprüchen 1 oder 2 in Kombination mit mindestens einem weiteren anorektischen Wirkstoff zur Anwendung als Medikament zur Behandlung von Störungen des Empfindens und anderer psychiatrischen Indikationen.

11. Verfahren zur Herstellung eines Arzneimittels enthaltend eine oder mehrere der Verbindungen gemäß den Ansprüchen 1 oder 2, **dadurch gekennzeichnet, daß** der Wirkstoff mit einem pharmazeutisch geeigneten Träger vermischt wird und diese Mischung in eine für die Verabreichung geeignete Form gebracht wird.

12. Verwendung der Verbindungen gemäß den Ansprüchen 1 oder 2 zur Herstellung eines Medikaments zur Gewichtsreduktion bei Säugetieren.

13. Verwendung der Verbindungen gemäß den Ansprüchen 1 oder 2 zur Herstellung eines Medikaments zur Prophylaxe oder Behandlung der Obesitas.

14. Verwendung der Verbindungen gemäß den Ansprüchen 1 oder 2 zur Herstellung eines Medikaments zur Prophylaxe oder Behandlung des Typ II Diabetes.

15. Verwendung der Verbindungen gemäß den Ansprüchen 1 oder 2 zur Herstellung eines Medikaments zur Behandlung von Störungen des Empfindens und anderer psychiatrischen Indikationen.

## Claims

1. A compound of the formula I 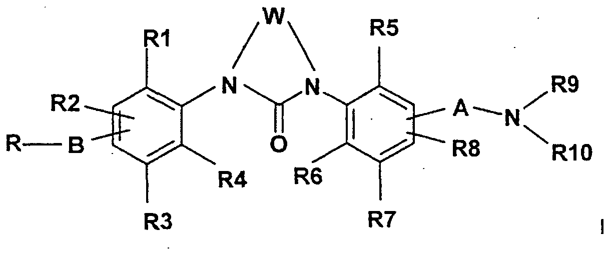 in which
R is (C₃-C₈)-cycloalkyl; 5-6 membered ring which may comprise one or two heteroatoms from the group of N, O and S, and the 5-6 membered ring may have further substituents selected from F, Cl, Br, NO₂, CF₃, OCF₃, CN, (C₁-C₆)-alkyl, O-(C₁-C₆)-alkyl;
B is 0, S, CO, OCH₂, N(R23), CH₂;
R23 is H, (C₁-C₆)-alkyl;
R1, R2, R3, R4 are, independently of one another, H, F, Cl, Br, CF₃, O-(C₁-C₆)-alkyl, (C₁-C₆)-alkyl;
W is -CH=CH-, -N=CH-;
R5, R6, R7, R8 are, independently of one another, H, F, Cl, Br, OH, CF₃, NO₂, CN, OCF₃, O-(C₁-C₆)-alkyl;
A is a chain -(C(R42) (R43))ₘ-, in which one member may be replaced by an element from the group of O, N(R44);
m is 3, 4;
R42, R43, R44 are, independently of one another, H, (C₁-C₆)-alkyl, aryl;
R9, R10 are, independently of one another, H, (C₁-C₈) -alkyl, - (CH₂) o-R45, CO-(C₁-C₈)-alkyl; or R9 and R10 form together with the nitrogen atom to which they are bonded a 4 to 10-membered mono-, bi- or spirocyclic ring which, apart from the nitrogen, may comprise 0 to 2 additional heteroatoms selected from the group of oxygen, nitrogen and sulfur, where the heterocyclic ring system may additionally be substituted by F, (C₁-C₆)-alkyl, O-(C₁-C₈)-alkyl, oxo, CO(R46), CON(R47)(R48), hydroxyl, N(R50)CO(C₁-C₆)-alkyl or N(R51)(R52);
R46, R47, R48, R50, R51, R52 are, independently of one another, H, (C₁-C₆)-alkyl;
o is 0, 1, 2, 3, 4;
R45 is OH, 5-10 membered mono- or bicyclic ring which may comprise one or two heteroatoms from the group of N, O and S, and the 5-10 membered ring may comprise further substituents selected from F, Cl, Br, OH, CF₃, oxo, O-(C₁-C₆)-alkyl, (C₁-C₆)-alkyl, (C₀-C₂)-alkylene-aryl, O-(C₀-C₂)-alkylene-aryl, or N(R51)(R52); and the physiologically tolerated salts thereof, where the alkyl, alkenyl and alkynyl radicals in the substituents R, R1, R2, R3, R4, R5, R6, R7, R8, R9, R10, R23, R42, R43, R44, R45, R46, R47, R48, R50, R51 and R52 may be either straight-chain, branched or optionally halogenated.

2. A compound of the formula I as claimed in claim 1, in which
W is -C=C-.

3. A medicament comprising one or more of the compounds as claimed in claim 1 or 2.

4. A medicament comprising one or more of the compounds as claimed in claim 1 or 2 and one or more anorectic active ingredients.

5. A compound as claimed in claim 1 or 2 for use as medicament for the prophylaxis or treatment of obesity.

6. A compound as claimed in claim 1 or 2 for use as medicament for the prophylaxis or treatment of type II diabetes.

7. A compound as claimed in claims 1 to 4 for use as medicament for the treatment of disturbances of wellbeing and other psychiatric indications.

8. A compound as claimed in claim 1 or 2 in combination with at least one other anorectic active ingredient for use as medicament for the prophylaxis or treatment of obesity.

9. A compound as claimed in claim 1 or 2 in combination with at least one other anorectic active ingredient for use as medicament for the prophylaxis or treatment of the type II diabetes.

10. A compound as claimed in claim 1 or 2 in combination with at least one other anorectic active ingredient for use as medicament for the treatment of disturbances of wellbeing and other psychiatric indications.

11. A process for producing a medicament comprising one or more of the compounds as claimed in claim 1 or 2, which comprises mixing the active ingredient with a pharmaceutically suitable carrier and converting this mixture into a form suitable for administration.

12. The use of the compound as claimed in claim 1 or 2 for producing a medicament for weight reduction in mammals.

13. The use of the compound as claimed in claim 1 or 2 for producing a medicament for the prophylaxis or treatment of obesity.

14. The use of the compound as claimed in claim 1 or 2 for producing a medicament for the prophylaxis or treatment of type II diabetes.

15. The use of the compound as claimed in claim 1 or 2 for producing a medicament for the treatment of disturbances of wellbeing and other psychiatric indications.

## Revendications

1. Composés de formule I 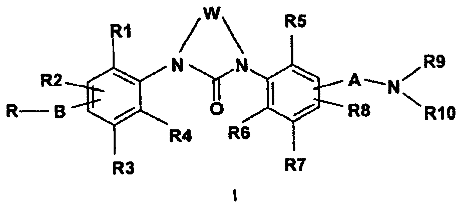 dans laquelle
R est un radical cycloalkyle en C₃-C₈ ;
un noyau à 5-6 chaînons, qui peut contenir un ou deux hétéroatomes du groupe N, O et S, et le noyau à 5-6 chaînons pouvant porter des substituants supplémentaires choisis parmi les substituants F, Cl, Br, NO₂, CF₃, OCF₃, CN, alkyle en C₁-C₆, O- (alkyle en C₁-C₆) ;
B est O, S, CO, OCH₂, N(R23), CH₂ ;
R23 est H, un radical alkyle en C₁-C₆ ;
R1, R2, R3, R4 représentent chacun indépendamment des autres H, F, Cl, Br, CF₃, un radical O-(alkyle en C₁-C₆), alkyle en C₁-C₆ ;
W est -CH=CH-, -N=CH- ;
R5, R6, R7, R8 représentent chacun indépendamment des autres H, F, Cl, Br, OH, CF₃, NO₂, CN, OCF₃, un radical O- (alkyle en C₁-C₆) ;
A est une chaîne -(C(R42) (R43))ₘ-, dans laquelle un chaînon peut être remplacé par un élément du groupe consistant en O, N(R44) ;
m vaut 3, 4 ;
R42, R43, R44 représentent chacun indépendamment des autres H, un radical alkyle en C₁-C₆, aryle ;
R9, R10 représentent chacun indépendamment de l'autre H, un radical alkyle en C₁-C₈, -(CH₂)ₒ-R45, CO-(alkyle en C₁-C₈) ; ou R9 et R10 forment, avec l'atome d'azote auquel ils sont liés, un noyau mono-, bi- ou spirocyclique à 4 à 10 chaînons, qui outre l'atome d'azote peut contenir 0 à 2 hétéroatomes supplémentaires choisis dans le groupe consistant en l'oxygène, l'azote et le soufre, le système cyclique hétérocyclique pouvant en outre être substitué par un ou des substituants F, alkyle en C₁-C₆, O- (alkyle en C₁-C₈), oxo, CO(R46), CON(R47)(R48), hydroxy, N(R50)CO(alkyle en C₁-C₆) ou N(R51) (R52) ;
R46, R47, R48, R50, R51, R52 représentent chacun indépendamment des autres H, un radical alkyle en C₁-C₆ ;
o vaut 0, 1, 2, 3, 4 ;
R45 est OH, un noyau mono- ou bicyclique à 5 à 10 chaînons, qui peut contenir un ou deux hétéroatomes du groupe consistant en N, O et S, et le noyau à 5-10 chaînons peut contenir des substituants supplémentaires choisis parmi les substituants F, Cl, Br, OH, CF₃, oxo, O- (alkyle en C₁-C₆), alkyle en C₁-C₆, (alkylène en C₀-C₂) -aryle, O-(alkylène en C₀-C₂)-aryle, ou N(R51)(R52) ; ou leurs sels physiologiquement compatibles,
les radicaux alkyle, alcényle et alcynyle, dans les substituants R, R1, R2, R3, R4, R5, R6, R7, R8, R9, R10, R23, R42, R43, R44, R45, R46, R47, R48, R50, R51 et R52, pouvant tant avoir une chaîne droite ou ramifiée, qu'être en option halogénés.

2. Composés de formule I selon la revendication 1, dans lesquels W est -C=C-.

3. Médicament contenant un ou plusieurs composés selon les revendications 1 ou 2.

4. Médicament contenant un ou plusieurs des composés selon les revendications 1 ou 2 et un ou plusieurs principes actifs anorexigènes.

5. Composés selon les revendications 1 ou 2, pour utilisation en tant que médicament pour la prophylaxie ou le traitement de l'obésité.

6. Composés selon les revendications 1 ou 2, pour utilisation en tant que médicament pour la prophylaxie ou le traitement du diabète de type II.

7. Composés selon les revendications 1 à 4 pour utilisation en tant que médicament pour le traitement de troubles de la sensibilité et d'autres indications psychiatriques.

8. Composés selon les revendications 1 ou 2, en combinaison avec au moins un autre principe actif anorexigène, pour utilisation en tant que médicament pour la prophylaxie ou le traitement de l'obésité.

9. Composés selon les revendications 1 ou 2, en combinaison avec au moins un autre principe actif anorexigène, pour utilisation en tant que médicament pour la prophylaxie ou le traitement du diabète de type II.

10. Composés selon les revendications 1 ou 2, en combinaison avec au moins un autre principe actif anorexigène, pour utilisation en tant que médicament pour le traitement de troubles de la sensibilité et d'autres indications psychiatriques.

11. Procédé de préparation d'un médicament contenant un ou plusieurs des composés selon les revendications 1 ou 2, **caractérisé en ce qu'**on mélange le principe actif à un support pharmaceutiquement approprié, et on met ce mélange sous une forme convenant à l'administration.

12. Utilisation des composés selon les revendications 1 ou 2 pour préparer un médicament pour la réduction du poids chez les mammifères.

13. Utilisation des composés selon les revendications 1 ou 2 pour préparer un médicament pour la prophylaxie ou le traitement de l'obésité.

14. Utilisation des composés selon les revendications 1 ou 2 pour préparer un médicament pour la prophylaxie ou le traitement du diabète de type II.

15. Utilisation des composés selon les revendications 1 ou 2 pour préparer un médicament pour le traitement de troubles de la sensibilité et d'autres indications psychiatriques.
